(19) Europäisches Patentamt
European Patent Office
Office européen des brevets

(11) EP 4 242 660 A1

# (12) EUROPEAN PATENT APPLICATION

(43) Date of publication:
**13.09.2023 Bulletin 2023/37**

(21) Application number: **23160420.8**

(22) Date of filing: **07.03.2023**

(51) International Patent Classification (IPC):
***G01N 33/58*** *(2006.01)* ***G01N 33/53*** *(2006.01)*

(52) Cooperative Patent Classification (CPC):
**G01N 33/582; G01N 33/5306**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.03.2022 JP 2022037188**

(71) Applicant: **SYSMEX CORPORATION**
**Kobe-shi**
**Hyogo 651-0073 (JP)**

(72) Inventors:
- **Kubo, Takuya**
**Kobe-shi, Hyogo, 651-0073 (JP)**
- **Suganuma, Masatoshi**
**Kobe-shi, Hyogo, 651-0073 (JP)**
- **Ishiki, Kengo**
**Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) REAGENT FOR MEASURING L-BIOTIN, METHOD FOR MEASURING SAMPLE CONTAINING L-BIOTIN, METHOD FOR DETERMINING NUMBER OF LABELS OF L-BIOTIN-LABELED SUBSTANCE, AND METHOD FOR PRODUCING SOLID PHASE ON WHICH OPTICALLY ISOMERIC BIOTIN-BINDING SITE IS IMMOBILIZED

(57) Disclosed is a reagent for measuring L-biotin, comprising an optically isomeric biotin-binding site and an azobenzene derivative represented by following formula (I):

[Chemical Formula 1]

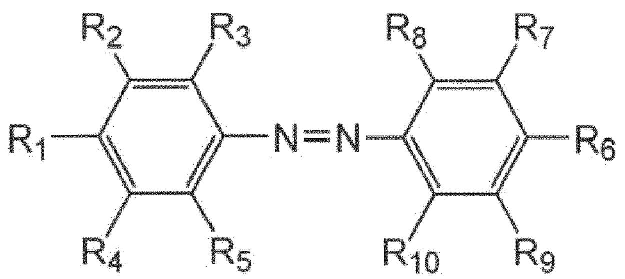

(I)

wherein $R_1$ to $R_{10}$ are each independently a group selected from the group consisting of a hydrogen atom, a hydroxy group, a carboxy group, C1 to C6 dialkylamino groups having no substituent or a substituted group, C1 to C6 alkyl groups having no substituent or a substituted group, and C1 to C6 alkoxy groups having no substituent or a substituted group, provided that at least one of $R_1$ to $R_5$ is a hydroxy group or a C1 to C6 dialkylamino group having no substituent or a substituted group, and at least one of $R_6$ to $R_{10}$ is a carboxy group.

EP 4 242 660 A1

FIG. 2

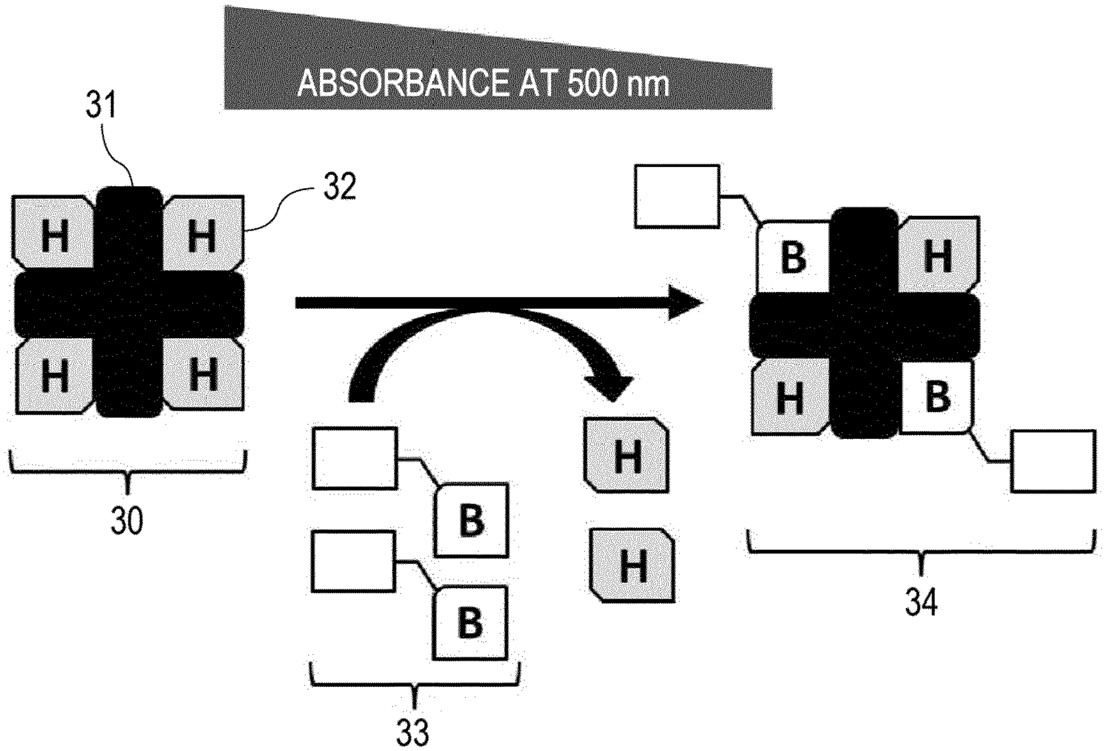

## Description

FIELD OF THE INVENTION

The present invention relates to a reagent for measuring L-biotin. The present invention relates to a method for measuring a sample containing L-biotin. The present invention relates to a method for determining the number of labels of an L-biotin-labeled substance. The present invention relates to a method for producing a solid phase on which an optically isomeric biotin-binding site is immobilized.

BACKGROUND

Since avidin and streptavidin have very high affinity with D-biotin, they are conventionally often utilized for immunological measurement. For example, when a target substance in a biological sample is measured using a D-biotin-labeled antibody and a solid phase on which avidin is immobilized, the target substance captured by the antibody is immobilized on the solid phase via binding between D-biotin and avidin. By detecting the target substance on the solid phase using, for example, a chemiluminescence method, the target substance can be measured with high sensitivity.

In an immunological measurement using the D-biotin-labeled antibody, the number of D-biotin bound to one molecule of antibody affects the measurement result. Therefore, it is necessary to quantify D-biotin bound to the antibody. For example, in Pierce (trademark) Biotin Quantitation Kit (product number: 28005), Thermo Fisher Scientific, it is described that a biotin quantification kit capable of measuring D-biotin bound to a protein such as an antibody or free D-biotin. The kit includes a reagent containing a mixture of 4'-hydroxyazobenzene-2-carboxylic acid (hereinafter, also referred to as "HABA") and avidin. The kit utilizes the fact that HABA and avidin bind to each other to form a complex having light absorption at a central wavelength of 500 nm, and that HABA in the complex is easily substituted with D-biotin to reduce absorption at 500 nm. Since the degree of reduction in absorption depends on the biotin concentration in a sample, in a method for measuring biotin using the kit, the biotin concentration is measured based on the absorbance at 500 nm. Such a method for measuring biotin is also called a HABA method.

SUMMARY OF THE INVENTION

In a biological sample such as blood, free D-biotin may be contained in the biological sample at a high concentration due to ingestion of a biotin-containing supplement or the like. Free D-biotin in the biological sample may compete with a D-biotin-labeled antibody to affect the measurement result. In order to solve such a problem, the present inventors have developed an immunological measurement method using a capture body labeled with L-biotin, which is an optical isomer of D-biotin, and a solid phase on which an optically isomeric biotin-binding site that does not substantially bind to D-biotin and binds to L-biotin is immobilized. On the other hand, also in the measurement method, it is necessary to control the number of L-biotin bound to one molecule of the capture body. However, a method and reagent capable of measuring L-biotin bound to a protein or the like and free L-biotin are not known. The reagent kit described in Pierce (trademark) Biotin Quantitation Kit (product number: 28005), Thermo Fisher Scientific, can also measure D-biotin, but cannot measure L-biotin. Thus, an object of the present invention is to provide a means enabling measurement of L-biotin.

The present inventors have found that L-biotin in a sample can be measured by a reagent containing an optically isomeric biotin-binding site and HABA in the same manner as in the HABA method, thereby completing the present invention. Therefore, the present invention provides a reagent for measuring L-biotin, containing an optically isomeric biotin-binding site and an azobenzene derivative represented by the following formula (I):

[Chemical Formula 1]

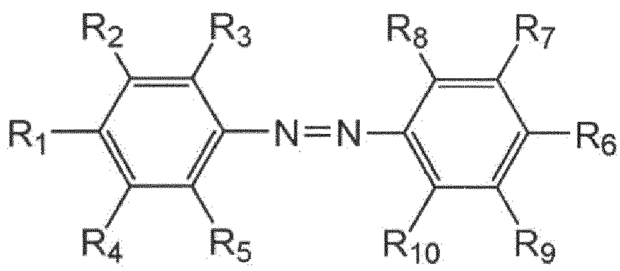

(I)

wherein $R_1$ to $R_{10}$ are each independently a group selected from the group consisting of a hydrogen atom, a hydroxy

group, a carboxy group, C1 to C6 dialkylamino groups having no substituent or a substituted group, C1 to C6 alkyl groups having no substituent or a substituted group, and C1 to C6 alkoxy groups having no substituent or a substituted group, provided that at least one of $R_1$ to $R_5$ is a hydroxy group or a C1 to C6 dialkylamino group having no substituent or a substituted group, and at least one of $R_6$ to $R_{10}$ is a carboxy group.

The present invention provides a method for measuring a sample containing L-biotin, including preparing a measurement sample by mixing a sample containing L-biotin with the reagent for measuring L-biotin; and measuring absorbance of the measurement sample, the measured value of absorbance being an index of concentration of L-biotin in the sample.

The present invention provides a method for determining the number of labels of an L-biotin-labeled substance, including preparing a measurement sample by mixing a sample containing an L-biotin-labeled substance with the reagent for measuring L-biotin; measuring absorbance of the measurement sample; determining a concentration of L-biotin in the sample based on the measured value of absorbance; and determining the number of L-biotin groups per molecule of the L-biotin-labeled substance based on the concentration of L-biotin in the sample and a concentration of the substance.

The present invention provides a method for producing a solid phase on which an optically isomeric biotin-binding site is immobilized, including preparing a measurement sample by mixing a sample separated from a liquid containing an L-biotin-labeled polypeptide with the reagent for measuring L-biotin; measuring absorbance of the measurement sample; determining a concentration of L-biotin in the measurement sample based on the measured value of absorbance; determining the number of L-biotin groups per molecule of the L-biotin-labeled polypeptide in the sample based on the concentration of L-biotin and the concentration of the polypeptide; when the number of L-biotin groups is within a predetermined range, contacting the liquid with a solid phase capable of binding to the polypeptide to immobilize the L-biotin-labeled polypeptide on the solid phase; and contacting the solid phase on which the L-biotin-labeled polypeptide is immobilized with an optically isomeric biotin-binding site to immobilize the optically isomeric biotin-binding site on the solid phase.

According to the present invention, a means capable of measuring L-biotin in a sample is provided.

## BRIEF DESCRIPTION OF THE DRAWINGS

Fig. 1A is a view showing an example of a reagent of the present embodiment in the form of one reagent.
Fig. 1B is a view showing an example of a reagent of the present embodiment in the form of two reagents.
Fig. 2 is a diagram showing a principle of L-biotin measurement using the reagent of the present embodiment.
Fig. 3 is a diagram showing an example of a solid phase on which an optically isomeric biotin-binding site is immobilized.
Fig. 4A is an example of a calibration curve prepared using Reagent 1 for measuring D-biotin in Example 1.
Fig. 4B is an example of a calibration curve prepared using Reagent 2 for measuring D-biotin in Example 1.
Fig. 4C is an example of a calibration curve prepared using a reagent for measuring L-biotin in Example 1.
Fig. 4D is an example of a calibration curve prepared using the reagent for measuring L-biotin in Example 1.
Fig. 5 is a graph showing a correlation between a measurement result by a reagent kit in Example 2 and a measurement result by a commercially available reagent kit.

## DETAILED DESCRIPTION OF THE PREFERRED EMBODIMENTS

The reagent for measuring L-biotin of the present embodiment (hereinafter, also referred to as "the reagent of the present embodiment") contains an optically isomeric biotin-binding site and an azobenzene derivative represented by the formula (I). The reagent of the present embodiment enables measurement of L-biotin according to the same principle as the HABA method. The optically isomeric biotin-binding site contained in the reagent of the present embodiment binds to L-biotin, but does not substantially bind to D-biotin. Therefore, even if D-biotin is mixed in the sample, an effect on the measurement result of L-biotin is suppressed.

As used herein, the "biotin-binding site" includes avidin and its analogs. Avidin and its analogs are polypeptides having high affinity with biotin and its analogs. As used herein, the "polypeptide" includes a protein and its fragments. The biotin-binding site may be deglycosylated polypeptide. Examples of the deglycosylated biotin-binding site include NeutrAvidin. NeutrAvidin is a deglycosylated avidin.

As used herein, the "biotin and its analogs" includes biotin and its analogs, and their optical isomers. Examples of the biotin analogs include desthiobiotin, biocytin, and the like. Each of biotin and its analogs has an optical isomer. For example, biotin has theoretically eight optical isomers. As used herein, the "L-biotin" includes free 3aR,4R,6aS-L-biotin, and a 3aR,4R,6aS-L-biotin group added to any substance. The term "D-biotin" includes free 3aS,4S,6aR-D-biotin,

and a 3aS,4S,6aR-D-biotin group added to any substance. The "biotin group" refers to a heterocyclic portion containing at least an imidazolidine ring in a chemical structure of biotin or its analog.

Examples of the avidin analogs include streptavidin, Pleurotus cornucopiae-derived avidin-like protein, bradavidin, rhizavidin, and the like. As used herein, the avidin analogs includes chimeras and variants of biotin-binding sites. The chimera refers to a modified polypeptide obtained by fusing all or a part of polypeptides constituting each of a plurality of kinds of biotin-binding sites. The variant refers to a modified polypeptide represented by an amino acid sequence in which at least one amino acid residue is substituted, deleted or added to the amino acid sequence of a predetermined biotin-binding site. The amino acid sequence of the variant is a polypeptide having 90% or more, preferably 95%, and more preferably 99% sequence identity as compared to the amino acid sequence of the original biotin-binding site.

As used herein, the avidin analogs includes optically isomeric biotin-binding sites. Generally, a biotin-binding site found in nature is an L-type biotin-binding site. In the present specification, the "L-type biotin-binding site" refers to a polypeptide, among the biotin-binding sites, in which amino acid residues other than glycine are composed of L-amino acid residues, that binds to D-biotin and does not substantially bind to L-biotin. In the present specification, the "optically isomeric biotin-binding site" refers to a polypeptide that has a part or all of an amino acid sequence of an L-type biotin-binding site, 90% or more of amino acid residues other than glycine in the amino acid sequence being D-amino acid residues, binds to L-biotin, and does not substantially bind to D-biotin. That is, the optically isomeric biotin-binding site contains the same amino acid sequence as the L-type biotin-binding site, but 90% or more of the amino acid residues constituting the polypeptide represented by the amino acid sequence are D-amino acid residues. Hereinafter, the optically isomeric biotin-binding site corresponding to a predetermined L-type biotin-binding site is also referred to as the "D-type polypeptide". In the present specification, the notation of "D-" and "L-" of the amino acid residue is based on the D/L notation.

In the optically isomeric biotin-binding site, 90% or more, for example, 90%, 91%, 92%, 93%, 94%, 95%, 96%, 97%, 98%, 99% or all of the amino acid residues other than glycine in the amino acid sequence of the L-type biotin-binding site may be D-amino acid residues. In a preferred embodiment, in the optically isomeric biotin-binding site, amino acid residues other than glycine in the amino acid sequence of the L-type biotin-binding site are composed of D-amino acid residues.

The biotin-binding site has a binding site with biotin or its analog. Hereinafter, the amino acid sequence of the polypeptide constituting the binding site with biotin or its analog in the biotin-binding site is also referred to as the "core sequence". In a preferred embodiment, the optically isomeric biotin-binding site has at least a core sequence of L-type biotin-binding site, and 90% or more of the amino acid residues other than glycine in the core sequence are D-amino acid residues. That is, the binding site with L-biotin in the optically isomeric biotin-binding site is composed of a polypeptide in which 90% or more of the amino acid residues other than glycine in the core sequence of L-type biotin-binding site are D-amino acid residues. Since 90% or more of the amino acid residues other than glycine are D-amino acid residues in the core sequence, it is considered that a steric structure of the binding site with L-biotin in the optically isomeric biotin-binding site is in an enantiomeric state with respect to the binding site with D-biotin in the L-type biotin-binding site. Therefore, it is considered that the optically isomeric biotin-binding site does not substantially bind to D-biotin and has high affinity for L-biotin.

The optically isomeric biotin-binding site has at least a core sequence of L-type biotin-binding site, and 90% or more, for example, 90%, 91%, 92%, 93%, 94%, 95%, 96%, 97%, 98%, and 99% of the amino acid residues other than glycine in the core sequence may be D-amino acid residues, or all of the amino acid residues other than glycine in the core sequence may be D-amino acid residues. In a preferred embodiment, the optically isomeric biotin-binding site has at least a core sequence of L-type biotin-binding site, and the amino acid residues other than glycine in the core sequence are composed of D-amino acid residues.

The optically isomeric biotin-binding site is a D-type polypeptide, and examples thereof include L-type biotin-binding sites such as streptavidin, avidin, Pleurotus cornucopiae-derived avidin-like protein, bradavidin, and zavidin, and D-type polypeptides corresponding to their chimeras and variants. The "corresponding D-type polypeptide" refers to a D-type polypeptide that has a part or all of an amino acid sequence of the L-type biotin-binding site and their chimeras and variants, 90% or more of the amino acid residues other than glycine in the amino acid sequence being D-amino acid residues, binds to L-biotin, and does not substantially bind to D-biotin. The D-type polypeptide corresponding to streptavidin (hereinafter, referred to as "the optically isomeric streptavidin") is a polypeptide containing a core sequence consisting of at least 19th to 133rd amino acid residues in the amino acid sequence of SEQ ID NO: 1, in which 90% or more of the amino acid residues other than glycine in the core sequence are D-amino acid residues. The amino acid sequence of SEQ ID NO: 1 is an amino acid sequence of streptavidin. Preferably, the optically isomeric streptavidin is a polypeptide containing a partial sequence consisting of at least 13th to 133rd amino acid residues in the amino acid sequence of SEQ ID NO: 1, in which 90% or more of the amino acid residues other than glycine in the partial sequence are D-amino acid residues. More preferably, the optically isomeric streptavidin is a polypeptide containing the amino acid sequence of SEQ ID NO: 1, in which 90% or more of the amino acid residues other than glycine in the amino acid

sequence are D-amino acid residues.

The D-type polypeptide corresponding to avidin (hereinafter, referred to as "optically isomeric avidin") is a polypeptide containing a core sequence consisting of at least 2nd to 128th amino acid residues in the amino acid sequence of SEQ ID NO: 2, in which 90% or more of the amino acid residues other than glycine in the core sequence are D-amino acid residues. The amino acid sequence of SEQ ID NO: 2 is an amino acid sequence of avidin. Preferably, the optically isomeric avidin is a polypeptide containing the amino acid sequence of SEQ ID NO: 2, in which 90% or more of the amino acid residues other than glycine in the amino acid sequence are D-amino acid residues.

Examples of the Pleurotus cornucopiae-derived avidin-like protein include tamavidin (registered trademark). Tamavidin (registered trademark) is an L-type biotin-binding site discovered from Pleurotus cornucopiae, and has high affinity for biotin and thermal stability superior to avidin (see WO 2002/072817). The D-type polypeptide corresponding to tamavidin (hereinafter, referred to as "optically isomeric tamavidin") (registered trademark) is a polypeptide containing a core sequence consisting of at least 4th to 129th amino acid residues in the amino acid sequence of SEQ ID NO: 3, in which 90% or more of the amino acid residues other than glycine in the core sequence are D-amino acid residues. The amino acid sequence of SEQ ID NO: 3 is an amino acid sequence of tamavidin (registered trademark) 1. Preferably, the optically isomeric avidin is a polypeptide containing the amino acid sequence of SEQ ID NO: 3, in which 90% or more of the amino acid residues other than glycine in the amino acid sequence are D-amino acid residues.

Alternatively, the optically isomeric tamavidin may be a polypeptide containing a core sequence consisting of at least 4th to 127th amino acid sequences of SEQ ID NO: 4, in which 90% or more of the amino acid residues other than glycine in the core sequence are D-amino acid residues. The amino acid sequence of SEQ ID NO: 4 is an amino acid sequence of tamavidin (registered trademark) 2. Preferably, the optically isomeric avidin is a polypeptide containing the amino acid sequence of SEQ ID NO: 4, in which 90% or more of the amino acid residues other than glycine in the amino acid sequence are D-amino acid residues.

An example of a streptavidin variant includes a variant described in Qureshi MH. et al., J. Biol. Chem., vol. 276, No. 49, pp. 46422-46428, 2001 (hereinafter, referred to as "streptavidin variant 1 "). The amino acid sequence of streptavidin variant 1 (SEQ ID NO: 5) has substitutions of S45A, T90A, and D128A as compared with the amino acid sequence of SEQ ID NO: 1. The D-type polypeptide corresponding to streptavidin variant 1 (hereinafter, referred to as "the optically isomeric streptavidin variant 1") is a polypeptide containing a core sequence consisting of at least 19th to 133rd amino acid residues in the amino acid sequence of SEQ ID NO: 5, in which 90% or more of the amino acid residues other than glycine in the core sequence are D-amino acid residues. Preferably, the optically isomeric streptavidin variant 1 is a polypeptide containing a partial sequence consisting of at least 13th to 133rd amino acid residues in the amino acid sequence of SEQ ID NO: 5, in which 90% or more of the amino acid residues other than glycine in the partial sequence are D-amino acid residues. More preferably, the optically isomeric streptavidin variant 1 is a polypeptide containing the amino acid sequence of SEQ ID NO: 5, in which 90% or more of the amino acid residues other than glycine in the amino acid sequence are D-amino acid residues.

Other examples of streptavidin variants include a variant described in Wu SC. and Wong SL., J. Biol. Chem., vol. 280, No. 24, pp. 23225-23231, 2005 (hereinafter, referred to as "streptavidin variant 2"). The amino acid sequence of streptavidin variant 2 (SEQ ID NO: 6) has substitutions of V55T, T76R, L109T, and V125R as compared with the amino acid sequence of SEQ ID NO: 1. The D-type polypeptide corresponding to streptavidin variant 2 (hereinafter, referred to as "the optically isomeric streptavidin variant 2") is a polypeptide containing a core sequence consisting of at least 19th to 133rd amino acid residues in the amino acid sequence of SEQ ID NO: 6, in which 90% or more of the amino acid residues other than glycine in the core sequence are D-amino acid residues. Preferably, the optically isomeric streptavidin variant 2 is a polypeptide containing a partial sequence consisting of at least 13th to 133rd amino acid residues in the amino acid sequence of SEQ ID NO: 6, in which 90% or more of the amino acid residues other than glycine in the partial sequence are D-amino acid residues. More preferably, the optically isomeric streptavidin variant 2 is a polypeptide containing the amino acid sequence of SEQ ID NO: 6, in which 90% or more of the amino acid residues other than glycine in the amino acid sequence are D-amino acid residues.

Other examples of streptavidin variants include a variant described in Lim KH. et al., Biotech. Bioeng., vol. 110, No. 1, pp. 57-67, 2013 (hereinafter, referred to as "streptavidin variant 3"). The D-type polypeptide corresponding to streptavidin variant 3 (hereinafter, referred to as "the optically isomeric streptavidin variant 3") is a polypeptide containing an amino acid sequence of SEQ ID NO: 7, in which 90% or more of the amino acid residues other than glycine in the amino acid sequence are D-amino acid residues. The amino acid sequence of SEQ ID NO: 7 is an amino acid sequence of streptavidin variant 3.

Other examples of streptavidin variants include a variant described in Sano T. et al., Proc. Natl. Acad. Sci. USA, vol. 94, pp. 6153-6158, 1997 (hereinafter, referred to as "streptavidin variant 4"). The amino acid sequence of streptavidin variant 4 (SEQ ID NO: 8) has a substitution of H127D and a deletion of G113 to W120 as compared with the amino acid sequence of SEQ ID NO: 1. The D-type polypeptide corresponding to streptavidin variant 4 (hereinafter, referred to as "the optically isomeric streptavidin variant 4") is a polypeptide containing a core sequence consisting of at least 19th to 125th amino acid residues in the amino acid sequence of SEQ ID NO: 8, in which 90% or more of the

amino acid residues other than glycine in the core sequence are D-amino acid residues. Preferably, the optically isomeric streptavidin variant 4 is a polypeptide containing a partial sequence consisting of at least 13th to 125th amino acid residues, at least 19th to 131st amino acid residues, or at least 13th to 131st amino acid residues in the amino acid sequence of SEQ ID NO: 8, in which 90% or more of the amino acid residues other than glycine in the partial sequence are D-amino acid residues. More preferably, the optically isomeric streptavidin variant 2 is a polypeptide containing the amino acid sequence of SEQ ID NO: 8, in which 90% or more of the amino acid residues other than glycine in the amino acid sequence are D-amino acid residues.

Other examples of streptavidin variants include a variant described in WO 2006/058226 (hereinafter, referred to as "streptavidin variant 5"). The D-type polypeptide corresponding to streptavidin variant 5 (hereinafter, referred to as "the optically isomeric streptavidin variant 5") is a polypeptide containing a core sequence consisting of at least 1st to 20th, 35th to 196th and 213rd to 261st amino acid sequences of SEQ ID NO: 9, in which 90% or more of the amino acid residues other than glycine in the core sequence are D-amino acid residues. The amino acid sequence of SEQ ID NO: 9 is an amino acid sequence of streptavidin variant 5. Preferably, the optically isomeric streptavidin variant 5 is a polypeptide containing a partial sequence consisting of at least 1st to 24th, 29 to 202nd and 207th to 261st amino acid residues in the amino acid sequence of SEQ ID NO: 9, in which 90% or more of the amino acid residues other than glycine in the partial sequence are D-amino acid residues. More preferably, the optically isomeric streptavidin variant 5 is a polypeptide containing the amino acid sequence of SEQ ID NO: 9, in which 90% or more of the amino acid residues other than glycine in the amino acid sequence are D-amino acid residues.

A biotin-binding site, including an optically isomeric biotin-binding site, generally exists in a form of a multimer composed of a plurality of subunits. The multimer may be, for example, a dimer, a tetramer or an octamer. The multimer is formed by association of a plurality of molecules of polypeptides of a predetermined biotin-binding site. For example, when the reagent of the present embodiment contains optically isomeric avidin or optically isomeric streptavidin, tetrameric optically isomeric avidin or optically isomeric streptavidin may be present in the reagent. The tetrameric optically isomeric avidin or optically isomeric streptavidin can bind to four L-biotins.

The optically isomeric biotin-binding site can be produced by a known method for synthesizing a polypeptide. The method for synthesizing a polypeptide is not particularly limited, and examples thereof include liquid phase synthesis, solid phase synthesis, synthesis by a cell-free system using artificial tRNA, and the like. When the number of amino acid residues of a polypeptide product is a certain number or more (generally 30 residues or more), a polypeptide can be produced by synthesizing two or more peptide fragments and then linking the polypeptides using a known ligation reaction.

The optically isomeric biotin-binding site can be synthesized, for example, by the following solid phase synthesis method.

(1) A carboxy group of a first amino acid residue protecting a nitrogen atom of an amino group is bound to a resin using a protecting group.
(2) The protecting group of the reactant obtained by the binding reaction of the above (1) is eliminated using a deprotecting agent, and then washed with a solvent to form a free amino acid.
(3) The free amino acid obtained in the above (2) is condensed with any amino acid having an amino group protected by the protecting group using a condensing agent.
(4) The protecting group of the product of the above (3) is eliminated using a deprotecting agent to form a free amino acid.
(5) By repeating the steps (2) to (4), it is possible to obtain a polypeptide to which an arbitrary amino acid to which a resin is bound is linked at the C-terminus.
(6) Washing is performed using a solvent at an arbitrary time point after the step (5) and at a time point when a resin to which a desired polypeptide is bound is produced.
(7) After the N-terminal amino group of the polypeptide to which the resin washed in the above (6) is bound is protected by a protecting group, the resin is cleaved using an acid, whereby any polypeptide to which the protecting group is bound can be obtained.

The resin used in the above (1) may be a known resin used in a solid phase synthesis method. As the resin that supplies the C-terminus as an amide group, for example, Rink-Amide-resin (manufactured by Merck KGaA), Rink-Amide-PEGA-resin (manufactured by Merck KGaA), and Fmoc-NH-SAL-resin (manufactured by WATANABE CHEMICAL INDUSTRIES, LTD.) functionalized with an amino group are preferably used. Fmoc-NH-SAL-resin-linker (manufactured by WATANABE CHEMICAL INDUSTRIES, LTD.) or the like may be bonded to AMINO-PEGA-resin (manufactured by Merck KGaA) functionalized with an amino group or the like.

As the resin for forming a carboxylic acid at the C-terminus, for example, 2-chlorotrityl chloride resin functionalized with chlorine (manufactured by Merck KGaA), AMINO-PEGA-resin functionalized with an amino group (manufactured by Merck KGaA), NovaSyn TGT alcohol resin having a hydroxy group (manufactured by Merck KGaA), Wang-resin (manufactured by Merck KGaA), HMPA-PEGA-resin (manufactured by Merck KGaA), and the like can be used.

A linker may be present between the AMINO-PEGA-resin and the amino acid, and examples of such a linker include 4-hydroxymethylphenoxyacetic acid (HMPA), 4-(4-hydroxymethyl-3-methoxyphenoxy)-butylacetic acid (HMPB), and the like. H-Cys(Trt)-TritylNovaPEG resin (manufactured by Merck KGaA) in which a C-terminal amino acid is previously bonded to a resin or the like can be used.

When a resin having a hydroxy group or a resin functionalized with chlorine is used, a bond between the resin and an amino acid in which a nitrogen atom of an amino group is protected by a protecting group bonds a carboxyl group of the amino acid to the resin by an ester bond. When a resin functionalized with an amino group is used, the carboxyl group of the amino acid is bonded to the resin by an amide bond.

The protecting group may be any known protecting group, and for example, carbonate-based or amide-based protecting groups such as a 9-fluorenylmethoxycarbonyl (Fmoc) group, a t-butyloxycarbonyl (Boc) group, a benzyl group, an allyloxycarbonyl group, and an acetyl group can be used. When a protecting group is introduced into an amino acid, for example, in the case of introducing an Fmoc group, the protecting group can be introduced by adding 9-fluorenylmethoxycarbonyl-N-succinimidyl carbonate and sodium carbonate and performing a reaction. The reaction temperature is 0 to 50°C, and preferably room temperature, and the reaction time is 1 to 5 hours, and preferably 3 hours.

As the amino acid in which the nitrogen atom of the amino group is protected using the protecting group, a commercially available amino acid may be used. Examples thereof include Fmoc-Ser-OH, Fmoc-Asn-OH, Fmoc-Val-OH, Fmoc-Leu-OH, Fmoc-Ile-OH, Fmoc-Ala-OH, Fmoc-Tyr-OH, Fmoc-Gly-OH, Fmoc-Lys-OH, Fmoc-Arg-OH, Fmoc-His-OH, Fmoc-Asp-OH, Fmoc-Glu-OH, Fmoc-Gln-OH, Fmoc-Thr-OH, Fmoc-Cys-OH, Fmoc-Met-OH, Fmoc-Phe-OH, Fmoc-Trp-OH, Fmoc-Pro-OH, Fmoc-SeMet-OH, and Fmoc-3-(methylseleno)-Ala-OH.

An amino acid protected by a protecting group and having a protecting group introduced into a side chain may be used. Examples thereof include Fmoc-Arg(Pbf)-OH, Fmoc-Asn(Trt)-OH, Fmoc-Asp(tBu)-OH, Fmoc-Cys(Acm)-OH, Fmoc-Cys(tBu)-OH, Fmoc-Cys(tBu)-OH, Fmoc-Cys(Trt)-OH, Fmoc-Glu(tBu)-OH, Fmoc-Gln(Trt)-OH, Fmoc-His(Trt)-OH, Fmoc-Lys(Boc)-OH, Fmoc-Ser(tBu)-OH, Fmoc-Thr(tBu)-OH, Fmoc-Trp(Boc)-OH, Fmoc-Tyr(tBu)-OH, Fmoc-Sec(Trt)-OH, Fmoc-Sec(pMeOBzl)-OH, Fmoc-Sec(pMeBzl)-OH, Fmoc-HomoSec(pMeBzl)-OH, and Fmoc-HomoSec(Mob)-OH.

When a resin having a hydroxy group is used, for example, an HMPB resin can be used as an esterification catalyst. At that time, as the dehydration condensing agent, a known dehydration condensing agent such as 1-mesitylenesulfonyl-3-nitro-1,2,4-triazole (MSNT), dicyclohexylcarbodiimide (DCC), or diisopropylcarbodiimide (DIC) can be used. As for the use ratio of the amino acid and the dehydration condensing agent, the dehydration condensing agent is used in an amount of usually 1 to 10 equivalents and preferably 1 to 5 equivalents based on 1 equivalent of the amino acid.

The esterification reaction is preferably carried out by, for example, subjecting a resin to a solid phase column, washing with a solvent, and adding an amino acid solution. Examples of the washing solvent include dimethylformamide (DMF), 2-propanol, dichloromethane (DCM), and the like. Examples of the solvent for dissolving an amino acid include dimethyl sulfoxide (DMSO), DMF, DCM, and the like. The reaction temperature of the esterification reaction is 0 to 50°C, and preferably room temperature. The reaction time is 10 minutes to 30 hours, and preferably 15 minutes to 24 hours. At this time, it is preferable to acetylate and cap the unreacted functional group on the solid phase using acetic anhydride or the like.

The lipid-soluble protecting group can be eliminated by, for example, a treatment with a base. Examples of the base include piperidine, morpholine, and the like. The treatment with a base is preferably performed in the presence of a solvent. Examples of the solvent include DMF, DMSO, methanol, and the like.

An amidation reaction between a free amino group and a carboxy group of any amino acid in which the amino group nitrogen is protected by the protecting group is preferably carried out in the presence of an activating agent, a base, and a solvent.

Examples of the activating agent include DIC, DCC, 1-ethyl-3-(3-dimethylaminopropyl)carbodiimide hydrochloride (WSC · HCl), diphenyl phosphoryl azide (DPPA), carbonyl diimidazole (CDI), diethyl cyanophosphonate (DEPC), benzotriazol-1-yloxy-trispyrrolidinophosphonium hexafluorophosphate (PyBOP), 1-hydroxybenzotriazole (HOBt), hydroxysuccinimide (HOSu), dimethylaminopyridine (DMAP), 1-hydroxy-7-azabenzotriazole (HOAt), hydroxyphthalimide (HOPht), pentafluorophenol (Pfp-OH), O-(1H-6-chlorobenzotriazol-1-yl)-1,1,3,3-tetramethyluronium hexafluorophosphate (HCTU), O-(7-azabenzotriazol-1-yl)-1,1,3,3-tetramethyluronium hexafluorophosphonate (HATU), 2-(1H-benzotriazol-1-yl)-1,1,3,3-tetramethyluronium tetrafluoroborate (TBTU), 3,4-dihydro-3-hydroxy-4-oxo-1,2,3-benzotriazine (DHBT), 4-(4,6-dimethoxy-1,3,5-triazin-2-yl)-4-methylmorpholinium chloride (DMT-MM), and the like.

The amount of the activating agent used is 1 to 20 equivalents, preferably 1 to 10 equivalents, and more preferably 1 to 5 equivalents with respect to any amino acid having an amino group nitrogen protected by a protecting group.

As the base, a base that can coexist with an alkylation reaction is preferable. Examples thereof include, but are not particularly limited to, N-ethyldiisopropylamine (DIPEA), 1,8-diazabicyclo[5.4.0]undec-7-ene (DBU), DMAP, 1,4-diazabicyclo[2.2.2]octane (DABCO), 2,6-dimethylpyridine, triethylamine (TEA), 1,5-diazabicyclo[4.3.0]nona-5-ene

(DBN), and the like.

Examples of the solvent include DMF, DMSO, DCM, and the like. The reaction temperature is 0 to 50°C, and preferably room temperature. The reaction time is 10 minutes to 30 hours, and preferably 15 minutes to 24 hours. The elimination of the protecting group can be performed in the same manner as described above. In order to cleave the peptide chain from the resin, it is preferable to treat with an acid. Examples of the acid include trifluoroacetic acid (TFA).

As a known ligation reaction for linking two or more peptide fragments, native chemical ligation (NCL method) (Dawson PE. et al., Science, vol. 266, pp. 776-779, 1994) can be used. The NCL method is a chemoselective reaction between a first peptide having an $\alpha$-carboxythioester moiety at the C-terminus and a second peptide having a cysteine residue at the N-terminus, a thiol group (also referred to as an SH group or a sulfhydryl group) of the cysteine side chain selectively reacts with carbonyl carbon of a thioester group, and a thioester binding initial intermediate is produced by a thiol exchange reaction. This intermediate spontaneously undergoes intramolecular rearrangement to give a native amide bond at the ligation site while regenerating a cysteine side-chain thiol.

In the NCL method, a cysteine binding site of the second peptide having a cysteine residue at the N-terminus can also be substituted with alanine by a desulfurization reaction (Yan LZ. and Dawson PE., J. Am. Chem. Soc., vol. 123, pp. 526-533, 2001) after the ligation reaction. That is, a site that is originally alanine is substituted with cysteine to perform synthesis, and can be used as a binding site for the ligation reaction.

Steps before and after the peptide synthesis reaction or the ligation reaction may include a separation and/or purification step. As the purification method, a known method may be used, and examples thereof include column chromatography and the like. Examples of the column chromatography include normal phase chromatography, reverse phase chromatography, gel filtration chromatography, affinity chromatography, and the like. A solvent, a filler of a column, a method for detecting an object to be separated and an object to be purified, a temperature condition, a pressure condition, and the like can be appropriately selected depending on the object to be separated and purified.

Known washing, drying, dilution, concentration steps, and the like may be appropriately included before and after the peptide synthesis reaction, ligation reaction, or separation and/or purification step.

When the polypeptide is not folded correctly, it is preferred to refold the polypeptide. The refolding can be performed by, for example, a dilution refolding method, a dialysis refolding method, a solid phase refolding method, a size exclusion chromatography refolding method, a surfactant refolding method, or the like (Arakawa T. and Ejima D., Antibodies, vol. 3, pp. 232-241, 2014).

The azobenzene derivative represented by the formula (I) is a dye capable of binding to a binding site with L-biotin of an optically isomeric biotin-binding site. When the optically isomeric biotin-binding site is present in the form of a multimer in the reagent of the present embodiment, a plurality of molecules of azobenzene derivatives bind to a binding site with L-biotin of a multimer of the optically isomeric biotin-binding site. It is considered that by binding to the multimer of the azobenzene derivative and the optically isomeric biotin-binding site, a complex of the azobenzene derivative and the optically isomeric biotin-binding site is formed in the reagent of the present embodiment. Due to the presence of the azobenzene derivative represented by the formula (I), the complex has light absorption at a wavelength of about 400 nm to about 600 nm at a pH near neutrality (pH 6.5 to 7.5).

In the present specification, the "C1 to C6 alkyl group" refers to a monovalent group that is a linear or branched saturated hydrocarbon chain having 1 to 6 carbon atoms. Examples of the C1 to C6 alkyl include, but are not limited to, methyl, ethyl, 1-propyl, 2-propyl, 1-butyl, 2-methyl-1-propyl, 2-butyl, 2-methyl-2-propyl (t-butyl), 1-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, and hexyl.

In the present specification, the "C1 to C6 alkoxy group" is a group in which the above-mentioned C1 to C6 alkyl is bonded to an oxy group. Examples of the C1 to C6 alkoxy group include a methoxy group, an ethoxy group, an n-propoxy group, an isopropoxy group, an n-butoxy group, a tert-butoxy group, an isobutoxy group, a sec-butoxy group, an n-pentyloxy group, an isopentyloxy group, an n-hexyloxy group, and the like.

In the present specification, the "C1 to C6 dialkylamino group" means a group in which two of the above-mentioned C1 to C6 alkyl are replaced with two hydrogen atoms of an amino group. The two C1 to C6 alkyl groups may be the same or different. Examples of the C1 to C6 dialkylamino include dimethylamino, diethylamino, N,N-diisopropylamino, N-methyl-N-ethylamino, N-isopropyl-N-ethylamino, and the like. Preferably, the C1 to C6 dialkylamino group is dimethylamino or diethylamino.

Substituents of the C1 to C6 alkyl groups, the C1 to C6 alkoxy groups, and the C1 to C6 dialkylamino groups are each independently selected from a halogen atom, a cyano group, a nitro group, a carboxy group, a carbamoyl group, an amino group, and a hydroxy group. The halogen atom is fluorine, chlorine, bromine, or iodine.

In a preferred embodiment, in the formula (I), any one of $R_1$ to $R_5$ is a hydroxy group, or a dimethylamino group or diethylamino group having no substituent, and any one of $R_6$ to $R_{10}$ is a carboxy group.

Examples of the azobenzene derivative represented by the formula (I) include HABA, 4'-hydroxyazobenzene-4-carboxylic acid, 4'-dimethylaminoazobenzene-2-carboxylic acid, and the like. These azobenzene derivatives are shown

in the following formulas (II) to (IV), respectively. Among them, HABA is particularly preferable.

[Chemical Formula 2]

HABA

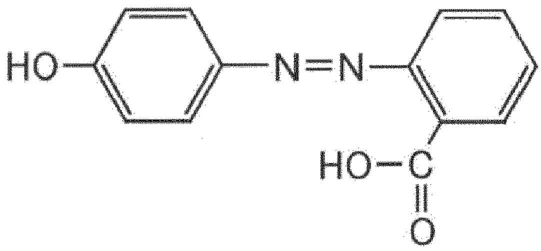

(II)

[Chemical Formula 3]

4'-Hydroxyazobenzene-4-carboxylic acid

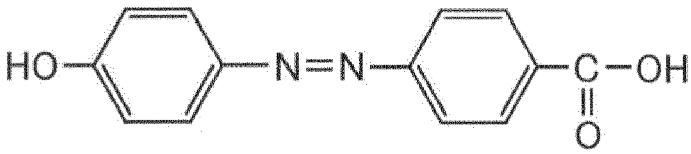

(III)

[Chemical Formula 4]

4'-Dimethylaminoazobenzene-2-carboxylic acid

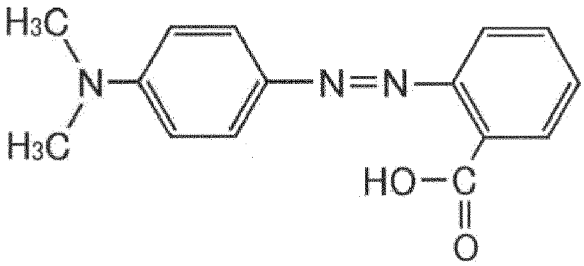

(IV)

The azobenzene derivative represented by the formula (I) can be obtained, for example, by a known synthesis method using a diazo coupling reaction or the like. Alternatively, a commercially available azobenzene derivative may be used. For example, as shown in the following reaction formula, anthranilic acid and sodium nitrite are reacted under cooling to produce a diazonium salt, and the diazonium salt and phenol are reacted, whereby HABA can be obtained.

[Chemical Formula 5]

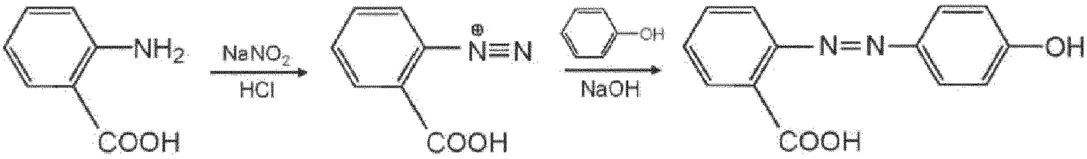


In the above reaction formula, when p-aminobenzoic acid is used in place of anthranilic acid, 4'-hydroxyazobenzene-4-carboxylic acid can be obtained. When dimethylaniline is used in place of phenol in the above reaction formula, 4'-dimethylaminoazobenzene-2-carboxylic acid can be obtained.

The reagent of the present embodiment can be prepared by dissolving an optically isomeric biotin-binding site and the azobenzene derivative represented by the formula (I) in an appropriate solvent. Alternatively, the reagent of the present embodiment can be prepared by mixing a solution of an optically isomeric biotin-binding site with a solution of the azobenzene derivative represented by the formula (I). The solvent is preferably an aqueous solvent having a pH near neutrality (pH 6.5 to 7.5), and examples thereof include water, physiological saline, a buffer solution having a buffering action at a pH near neutrality, and the like. Examples of the buffer solution include phosphate buffered saline (PBS), phosphate buffer, borate buffer, tris hydrochloride buffer, BES buffer, HEPES buffer, MOPS buffer, TES buffer, and the like.

The pH of the reagent of the present embodiment is usually 6.5 or more and 7.5 or less, and preferably 7 or more and 7.5 or less. The concentration of the optically isomeric biotin-binding site in the reagent of the present embodiment is not particularly limited, and is, for example, 50 μg/mL or more and 2000 μg/mL or less, preferably 100 μg/mL or more and 1000 μg/mL or less, and more preferably 200 μg/mL or more and 500 μg/mL or less. The concentration of the azobenzene derivative represented by the formula (I) in the reagent of the present embodiment is not particularly limited, and is, for example, 5 μg/mL or more and 200 μg/mL or less, preferably 10 μg/mL or more and 100 μg/mL or less, and more preferably 20 μg/mL or more and 50 μg/mL or less.

The reagent of the present embodiment may be in the form of one reagent containing both the optically isomeric biotin-binding site and the azobenzene derivative represented by the formula (I) in one reagent. Alternatively, the reagent of the present embodiment may be in the form of two reagents containing a first reagent containing the optically isomeric biotin-binding site and a second reagent containing the azobenzene derivative represented by the formula (I).

A container containing the reagent of the present embodiment may be packed in a box and provided to a user. The box may contain a package insert describing how to use the reagents and the like. Fig. 1A and Fig. 1B show an example of the reagent of the present embodiment. With reference to Fig. 1A, 10 denotes a reagent in the form of one reagent, 11 denotes a first container containing a reagent, 12 denotes a packing box, and 13 denotes a package insert. With reference to Fig. 1B, 20 denotes a reagent in the form of two reagents, 21 denotes a first container containing a first reagent, 22 denotes a second container containing a second reagent, 23 denotes a packing box, and 24 denotes a package insert.

The reagent of the present embodiment may contain the optically isomeric biotin-binding site and the azobenzene derivative represented by the formula (I) in a solid state (powder, particle, freeze-dried product, and the like). Alternatively, the reagent of the present embodiment may contain the optically isomeric biotin-binding site and the azobenzene derivative represented by the formula (I) in a solution state. When the reagent is in the form of two reagents, one of the optically isomeric biotin-binding site and the azobenzene derivative represented by the formula (I) may be in a solid state, and the other may be in a solution state. Preferably, both the optically isomeric biotin-binding site and the azobenzene derivative represented by the formula (I) are in a solution state.

The reagent of the present embodiment may further include a calibrator. The calibrator is a solution containing L-biotin at a predetermined concentration, and is used to prepare a calibration curve. The calibrator preferably includes a plurality of solutions with different L-biotin concentrations. The calibrator preferably includes a solution with an L-biotin concentration of 0, for example, a buffer solution containing no L-biotin.

Another embodiment is a method for measuring a sample containing L-biotin. Hereinafter, this method is also referred to as "the measurement method of the present embodiment". In the measurement method of the present embodiment, first, a sample containing L-biotin and the reagent of the present embodiment are mixed to prepare a measurement sample. The sample containing L-biotin is not particularly limited as long as it contains free L-biotin and/or a substance having at least one L-biotin group. In the measurement method of the present embodiment, the sample containing L-biotin is also referred to as "the test sample".

The substance having at least one L-biotin group may be, for example, an L-biotin-labeled substance. In the L-biotin-labeled substance, it is preferable that L-biotin and the substance are covalently bonded. The substance is not particularly limited, and examples thereof include polypeptides, polynucleotides, lipids, sugar chains, and the like. A preferred L-biotin-labeled substance is an L-biotin-labeled polypeptide. The type of polypeptide is not particularly limited, and examples thereof include antibodies, albumin, enzymes such as alkaline phosphatase, and the like.

In the present specification, the "antibody" also includes an antibody fragment. Examples of the antibody fragment include Fab, Fab', F(ab')2, and the like. The antibody may be either a monoclonal antibody or a polyclonal antibody. The origin of the antibody is not particularly limited, and may be an antibody derived from any mammal such as a mouse, a rat, a hamster, a rabbit, a goat, a horse, or a camel. The isotype of antibody may be any of IgG, IgM, IgE, IgA and the like and is preferably IgG.

The L-biotin labeling can be performed, for example, by reacting a substance with an L-biotin labeling reagent. The L-biotin labeling reagent can be obtained by inducing a valeric acid side chain of L-biotin by a known method and introducing a labeling functional group. The labeling functional group is not particularly limited, and examples thereof

include an N-hydroxysuccinimide (NHS) ester, a maleimide group, and the like.

The measurement method of the present embodiment is preferably carried out in a solution, and thus, when the test sample is not in a liquid state, it is preferable to previously prepare the test sample in a liquid form. The "liquid" sample is not limited to a solution in which a solute is completely dissolved in a solvent, but also includes a suspension in which fine solids are suspended, a sol, and the like. For example, when the test sample contains a solid L-biotin-labeled substance, the substance can be dissolved with an appropriate solvent to form a liquid sample. The solvent is the same as that described for the reagent of the present embodiment.

The mixing amount of the sample containing L-biotin and the reagent of the present embodiment and the final concentration of the reagent are not particularly limited, and can be appropriately determined. For example, in the measurement of the absorbance of the measurement sample described later, when the value of the absorbance is lower than a predetermined value, it is considered that the L-biotin concentration in the test sample is high. In this case, it is preferable to dilute the test sample and prepare the measurement sample again from the diluted test sample. The predetermined value can be appropriately determined, but for example, when the absorbance is measured using a cuvette, the predetermined value can be 0.3. When the absorbance is measured using a microplate, the predetermined value may be 0.15.

Conditions of temperature and time in mixing the sample containing L-biotin with the reagent of the present embodiment are not particularly limited. For example, the mixture is incubated at 4°C to 40°C, and preferably at room temperature (about 20°C) to 37°C, for 1 minute to 60 minutes, and preferably 5 minutes to 30 minutes. During the incubation, the mixture may be allowed to stand, or may be stirred or shaken.

In the measurement method of the present embodiment, the absorbance of the prepared measurement sample is measured. Specifically, the absorbance of the azobenzene derivative represented by the formula (I) at a measurable wavelength (for example, maximum absorption wavelength) is measured. Such a wavelength can be appropriately determined from a range of about 400 nm to about 600 nm. For example, when HABA is used as the azobenzene derivative, absorbance at a wavelength of 500 nm may be measured. When 4'-hydroxyazobenzene-4-carboxylic acid is used, the absorbance at a wavelength of 470 nm may be measured. When 4'-dimethylaminoazobenzene-2-carboxylic acid is used, absorbance at a wavelength of 548 nm or 577 nm may be measured.

With reference to Fig. 2, a principle of the measurement method of the present embodiment will be described. In the example shown in Fig. 2, the reagent of the present embodiment contains an optically isomeric biotin-binding site and HABA, and the test sample contains an L-biotin-labeled polypeptide, but the present invention is not limited to this example. In this reagent, a complex 30 of a tetramer 31 of an optically isomeric biotin-binding site and HABA32 is formed. The complex 30 has absorption at a wavelength of 500 nm due to the presence of HABA 32. When the reagent of the present embodiment is mixed with a sample containing an L-biotin-labeled polypeptide 33, the complex 30 and the polypeptide 33 come into contact with each other. By this contact, the HABA 32 in the complex 30 is substituted with the L-biotin group of the polypeptide 33, and the HABA 32 is released. This is because L-biotin has higher affinity for optically isomeric biotin-binding site than HABA. When the HABA 32 is substituted with the L-biotin group of the polypeptide 33, a complex 34 in which a part or all of HABA 32 bound to the tetramer 31 of the optically isomeric biotin-binding site is substituted with the polypeptide 33 is formed. The complex 34 has lower absorption at a wavelength of 500 nm than the complex 30. The degree of decrease in absorption depends on the concentration of L-biotin in the test sample. In the example shown in Fig. 2, the concentration corresponds to the number of L-biotin groups of all the polypeptides 33 in the test sample. Therefore, in the measurement method of the present embodiment, the measured value of absorbance is an index of the concentration of L-biotin in the sample containing L-biotin.

In the measurement method of the present embodiment, the L-biotin concentration in the sample containing L-biotin can be measured based on the measured value of absorbance. For example, a plurality of calibrators containing free L-biotin at a predetermined concentration are measured in the same manner as the test sample, and a calibration curve indicating a relationship between the measured value of absorbance and the L-biotin concentration is prepared. Using the obtained calibration curve, the value of the L-biotin concentration in the test sample can be determined from the measured value of absorbance of the test sample.

Another embodiment is a method for determining the number of labels of an L-biotin-labeled substance. Hereinafter, this method is also referred to as "the determination method of the present embodiment". In the present specification, "the number of labels of an L-biotin-labeled substance" refers to the number of L-biotin groups per molecule of the L-biotin-labeled substance. One or more L-biotin groups may be added to the substance depending on the kind of the substance or the L-biotin labeling reagent. For example, when the substance is an antibody, the number of L-biotin groups per molecule of the L-biotin-labeled antibody may contribute to the result of an assay using the L-biotin-labeled antibody. According to the determination method of the present embodiment, it is possible to control quality of the L-biotin-labeled antibody.

In the determination method of the present embodiment, first, a sample containing an L-biotin-labeled substance and the reagent of the present embodiment are mixed to prepare a measurement sample. Details of the preparation of the L-biotin-labeled substance and the measurement sample are the same as those described for the measurement

method of the present embodiment. In the determination method of the present embodiment, the sample containing an L-biotin-labeled substance is also referred to as "the test sample".

In the determination method of the present embodiment, the absorbance of the prepared measurement sample is measured. Then, the concentration of L-biotin in the test sample is determined based on the measured value of absorbance. Details of the measurement of the absorbance and the determination of L-biotin concentration are the same as those described for the measurement method of the present embodiment. The L-biotin concentration determined based on the measured value of absorbance corresponds to the number of L-biotin groups of the entire L-biotin-labeled substance in the test sample. Therefore, the number of L-biotin groups per molecule of the L-biotin-labeled substance can be determined by comparing the determined L-biotin concentration with the concentration of the substance in the test sample. That is, in the determination method of the present embodiment, the number of L-biotin groups per molecule of the L-biotin-labeled substance is determined based on the concentration of L-biotin and the concentration of the substance in the test sample.

The determination method of the present embodiment may further include determining the concentration of the substance in the test sample. The method for determining the concentration of the substance can be appropriately selected according to the kind of the substance. For example, when the L-biotin-labeled substance is an L-biotin-labeled polypeptide, the polypeptide concentration in the test sample can be determined by a known total protein quantification method. As the total protein quantification method, a quantification method based on absorbance is preferable, and examples thereof include an ultraviolet absorptiometry (280 nm method), a BCA method, a Bradford method, a Lowry method, a method using Pierce (trademark) 660 nm Protein Assay Kit (Thermo Fisher Scientific), and the like. When the L-biotin-labeled substance is an L-biotin-labeled polynucleotide, the polynucleotide concentration in the test sample can be determined by a known polynucleotide quantification method. Examples of the polynucleotide quantification method include an absorption analysis method based on absorbance at 260 nm and 280 nm, a fluorescence analysis method using a fluorescent dye or a fluorescent probe, and the like.

The number of L-biotin groups per molecule of the L-biotin-labeled substance corresponds to the ratio of the number of molecules of L-biotin to the number of molecules of the substance. Therefore, the concentration of L-biotin and the concentration of the substance in the test sample are preferably molar concentrations (the unit is mol/L, mol/$dm^3$ or M). When the concentration of the substance in the test sample is determined by a quantification method based on absorbance, it is preferable to calculate the molar concentration from the value of absorbance. For example, the absorbance of a standard solution of a substance having a known concentration is measured, and the molar concentration can be calculated from the absorbance of the substance of the test sample using the absorbance of the standard solution, the concentration of the substance, and the molecular weight of the substance.

In a preferred embodiment, the number of L-biotin groups per molecule of the L-biotin-labeled substance is calculated by the following formula.

$$X = A/B$$

wherein X is the number of L-biotin groups per molecule of the L-biotin-labeled substance,
A is a molar concentration of L-biotin in the test sample, and
B is a molar concentration of the substance in the test sample.

Another embodiment is a method for producing a solid phase on which an optically isomeric biotin-binding site is immobilized. Hereinafter, this method is also referred to as "the production method of the present embodiment". In the production method of the present embodiment, using a sample separated from a liquid containing an L-biotin-labeled polypeptide, the number of L-biotin groups per molecule of the L-biotin-labeled polypeptide in the liquid is determined. Then, when the determined number of L-biotin groups is within a predetermined range, a solid phase on which an optically isomeric biotin-binding site is immobilized is produced using the same liquid.

With reference to Fig. 3, a solid phase produced by the production method of the present embodiment will be described. In the example shown in Fig. 3, a solid phase 40 is a spherical particle, but the present invention is not limited thereto. The solid phase 40 is a particle whose surface is capable of binding to a polypeptide portion 41 of the L-biotin-labeled polypeptide. In the production method of the present embodiment, after the solid phase 40 is bound to the polypeptide portion 41 of the L-biotin-labeled polypeptide, an L-biotin group 42 of the L-biotin-labeled polypeptide is bound to an optically isomeric biotin-binding site 43. In the example shown in Fig. 3, the optically isomeric biotin-binding site 43 is a tetramer. In the example shown in Fig. 3, since the number of L-biotin groups per molecule of the L-biotin-labeled polypeptide is 4, four optically isomeric biotin-binding sites 43 are bound. As described above, Fig. 3 shows a solid phase on which a plurality of optically isomeric biotin-binding sites are immobilized via the bond between the L-biotin group of the L-biotin-labeled polypeptide immobilized on the solid phase and the optically isomeric biotin-binding site. However, the present invention is not limited to this example.

In the production method of the present embodiment, first, a sample separated from a liquid containing an L-biotin-labeled polypeptide and the reagent for measuring L-biotin of the present embodiment are mixed to prepare a measurement sample. In the production method of the present embodiment, the sample separated from a liquid containing an L-biotin-labeled polypeptide is also referred to as "the test sample". Details of the preparation of the L-biotin-labeled substance and the measurement sample are the same as those described for the measurement method of the present embodiment. The amount of the test sample is not particularly limited, and is preferably the minimum amount necessary for measuring the absorbance.

Next, the absorbance of the measurement sample is measured, and the concentration of L-biotin in the measurement sample is determined based on the measured value of absorbance. Details of the measurement of the absorbance and the determination of L-biotin concentration are the same as those described for the measurement method of the present embodiment. Then, the number of L-biotin groups per molecule of the L-biotin-labeled polypeptide in the test sample is determined based on the concentration of L-biotin and the concentration of the polypeptide. Details of the determination of the number of L-biotin groups are the same as those described for the determination method of the present embodiment.

The production method of the present embodiment may include determining whether or not the number of L-biotin groups per molecule of the L-biotin-labeled polypeptide in the test sample is within the predetermined range. In the production method of the present embodiment, as shown in Fig. 3, the optically isomeric biotin-binding site is immobilized on the solid phase via the bond between the L-biotin group of the L-biotin-labeled polypeptide and the optically isomeric biotin-binding site. The number of optically isomeric biotin-binding sites to be immobilized depends on the number of L-biotin groups of the L-biotin-labeled polypeptide. Therefore, the number of L-biotin groups affects quality of the solid phase obtained by the production method of the present embodiment.

In the production method of the present embodiment, when the number of L-biotin groups per molecule of the L-biotin-labeled polypeptide in the test sample is within the predetermined range, the liquid is contacted with a solid phase capable of binding to the polypeptide to immobilize the L-biotin-labeled polypeptide on the solid phase. All or a part of the liquid may be used. The contact conditions are not particularly limited, and for example, incubation may be carried out at 4°C to 40°C, and preferably room temperature (about 20°C) to 37°C, for 10 minutes to 4 hours, and preferably 30 minutes to 3 hours. When the solid phase is a particle, the mixture of the solid phase and the L-biotin-labeled polypeptide may be left to stand, stirred, or shaken during the incubation.

When the number of L-biotin groups per molecule of the L-biotin-labeled polypeptide in the test sample is not within the predetermined range, the L-biotin-labeled polypeptide is prepared again to prepare a new liquid. Alternatively, when there is a plurality of liquids, the number of L-biotin groups per molecule of the L-biotin-labeled polypeptide may be determined for another liquid.

The solid phase may be an insoluble carrier capable of binding to the polypeptide portion of the L-biotin-labeled polypeptide. A binding mode between the solid phase and the polypeptide portion of the L-biotin-labeled polypeptide is not particularly limited, and examples thereof include physical adsorption or covalent bonding to the surface of the solid phase. As the covalent bond between the solid phase surface and the polypeptide, for example, a functional group such as an NHS ester or a maleimide group can be imparted to the solid phase surface, and the polypeptide portion can be covalently bonded to the solid phase surface by the functional group. Alternatively, the polypeptide portion and the solid phase surface are covalently bonded with a bivalent crosslinking agent.

The material of the solid phase is not particularly limited. For example, the material can be selected from organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, and the like), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include a particle, a membrane, a microplate, a microtube, a test tube, and the like. Among them, a particle and a microplate are preferable. The particle is particularly preferably a magnetic particle.

After contacting the solid phase with the L-biotin-labeled polypeptide, B/F (Bound/Free) separation for removing unreacted free components may be performed before further contacting the optically isomeric biotin-binding site. The unreacted free component may be, for example, an L-biotin-labeled polypeptide that is not immobilized on the solid phase. When the solid phase is a particle, B/F separation can be performed by precipitating the particle by centrifugation and removing a supernatant containing an unreacted free component. When the solid phase is a magnetic particle, B/F separation can be performed, for example, by removing a liquid containing an unreacted free component while magnetically constraining the magnetic particle with a magnet. When the solid phase is a container such as a microplate, B/F separation can be performed by removing a liquid containing an unreacted free component from the container. After B/F separation, the solid phase on which the L-biotin-labeled polypeptide is immobilized may be washed with a suitable aqueous medium such as PBS.

In the production method of the present embodiment, the solid phase on which the L-biotin-labeled polypeptide

is immobilized is contacted with the optically isomeric biotin-binding site to immobilize the optically isomeric biotin-binding site on the solid phase. As shown in Fig. 3, the optically isomeric biotin-binding site is immobilized on the solid phase via the bond between the L-biotin group of the L-biotin-labeled polypeptide and the optically isomeric biotin-binding site. The contact conditions are not particularly limited, and for example, incubation may be carried out at 4°C to 40°C, and preferably room temperature (about 20°C) to 37°C, for 1 minute to 1 hour, and preferably 5 minutes to 30 minutes. When the solid phase is a particle, the mixture of the solid phase and the optically isomeric biotin-binding site may be left to stand, stirred, or shaken during the incubation. After the solid phase and the optically isomeric biotin-binding site are contacted with each other, B/F separation may be performed. By the contact between the solid phase and the optically isomeric biotin-binding site, a solid phase on which the optically isomeric biotin-binding site is immobilized can be obtained.

By combining the solid phase on which the optically isomeric biotin-binding site obtained by the production method of the present embodiment is immobilized with the L-biotin-labeled antibody, a reagent kit including a solid phase used for an enzyme-linked immunosorbent assay (ELISA) method and a capture body can be obtained.

Hereinbelow, the present invention will be described in detail by examples, but the present invention is not limited to these examples.

## EXAMPLES

### Example 1: Preparation and Use of Reagent for Measuring L-Biotin

A method is known in which a complex of a biotin-binding site and HABA has absorption at a wavelength of 500 nm, and biotin in a sample is measured by utilizing the fact that HABA in the complex is easily replaced with free or bound D-biotin. In the measurement method, whether or not L-biotin in a sample can be measured when an optically isomeric biotin-binding site is used in place of the biotin-binding site was examined. Specifically, a reagent for measuring L-biotin containing optically isomeric streptavidin and HABA was prepared, and measurement of the L-biotin-labeled polypeptide and determination of the number of biotin labels were performed using the reagent. For comparison, a reagent for measuring D-biotin was also prepared, and the same experiment was performed.

#### 1. Preparation of reagent for measuring biotin

#### (1.1) Preparation of reagent for measuring L-biotin

PBS was prepared by mixing 0.1 M phosphate buffer (pH 7.5, 200 mL) and sodium chloride (1.75 g). HABA (1.12 mg: Tokyo Chemical Industry Co., Ltd.) and PBS (20 mL) were mixed to obtain a HABA solution. Optically isomeric streptavidin was obtained by peptide synthesis by entrusting to GlyTech, Inc. In the obtained optically isomeric streptavidin, amino acid residues other than glycine were D-amino acid residues, and the amino acid sequence thereof was the same as that of the polypeptide consisting of amino acid sequences from 13th to 133rd positions of the amino acid sequence of SEQ ID NO: 1. Optically isomeric streptavidin (1.28 mg) was dissolved in PBS (2 mL) to obtain an optically isomeric streptavidin solution. The optically isomeric streptavidin solution (368 μL) and the HABA solution (432 μL) were mixed to prepare a reagent for measuring L-biotin. In the reagent, the concentration of the optically isomeric streptavidin was 294 μg/mL, and the concentration of HABA was 30 μg/mL.

#### (1.2) Preparation of Reagent 1 for measuring D-biotin

HABA (1 mg) and PBS (33 mL) were mixed to obtain a HABA solution. Avidin (1 mg: FUJIFILM Wako Pure Chemical Corporation) and a HABA solution (4 mL) were mixed to prepare Reagent 1 for measuring D-biotin. In the reagent, the concentration of avidin was 250 μg/mL, and the concentration of HABA was 30 μg/mL.

#### (1.3) Preparation of Reagent 2 for measuring D-biotin

HABA (1.12 mg) and PBS (20 mL) were mixed to obtain a HABA solution. Streptavidin (1.28 mg: Roche Diagnostics K.K.) and PBS (2 mL) were mixed to obtain a streptavidin solution. The streptavidin solution (368 μL) and the HABA solution (432 μL) were mixed to prepare Reagent 2 for measuring D-biotin. In the reagent, the concentration of the streptavidin was 194 μg/mL, and the concentration of HABA was 30 μg/mL.

2. Preparation of biotin standard solution as calibrator

(2.1) Preparation of L-biotin standard solution

L-biotin was obtained as follows. A mixture of D-biotin and L-biotin was synthesized by the method described in Lavielle S. et al., J. Am. Chem. Soc., vol. 100, pp. 1558-1563, 1978. The mixture was subjected to optical resolution by liquid chromatography by entrusting to Daicel Corporation to obtain L-biotin. CHIRALPAK (registered trademark) IG (Φ 46 × 50 mm: Daicel Corporation) was used as a column, and a mixed solvent of methanol and acetic acid (100 : 0.1 (v/v)) was used as a mobile phase. The optical resolution was performed under the conditions of a flow rate of 1.0 mL/min, a column temperature of 40°C, and a detection wavelength of 205 nm. The obtained L-biotin (1 mg) and 0.1 M phosphate buffer (pH 7.5, 40.9 mL) were mixed to prepare a 100 μM L-biotin standard solution. The standard solution was diluted with 0.1 M phosphate buffer (pH 7.5) to further prepare 25 μM and 50 μM L-biotin standard solutions.

(2.2) Preparation of D-biotin standard solution

D-Biotin (1 mg: Kishida Chemical Co., Ltd.) and 0.1 M phosphate buffer (pH 7.5, 40.9 mL) were mixed to prepare a 100 μM D-biotin standard solution. The standard solution was diluted with 0.1 M phosphate buffer (pH 7.5) to further prepare 25 μM and 50 μM D-biotin standard solutions.

3. Preparation of sample

(3.1) Preparation of L-biotin labeling reagent

In order to prepare an L-biotin-labeled polypeptide, L-biotin (L-biotin-AC5-NHS) to which N-hydroxysuccinimide (NHS) ester was added and L-biotin (L-biotin-PE-maleimide) to which a maleimide group was added were synthesized as follows, using the L-biotin obtained in the above (2.1). D-Biotin-AC5-NHS and D-biotin-PE-maleimide were purchased from DOJINDO LABORATORIES (Biotin-AC5-OSu: product code B305, Biotin-PEAC5-maleimide: product code B299). The notation of the compound name and NHS in the chemical formula in the present specification is synonymous with OSu.

(3.1.1) Synthesis ofL-biotin-AC5-NHS

2,5-Dioxopyrrolidin-1-yl 5-((3aR,4R,6aS)-2-oxohexahydro-1H-thieno[3,4-d]imidazol-4-yl) pentanoic acid ester (Compound 2) was synthesized using L-biotin (Compound 1) according to the following synthesis scheme. Specifically, the synthesis was as follows. L-Biotin (123 mg, 0.503 mmol) and NHS (69.4 mg, 0.603 mmol) were dissolved in N,N-dimethylformamide (DMF) (3.4 mL), ethyl(dimethylaminopropyl)carboxydiimide (EDC) (116 mg, 0.603 mmol) was added thereto, and the mixture was stirred at room temperature (rt) for 24 hours. The solvent was evaporated and the resulting residue recrystallized from ethanol : acetic acid : water (95 : 1 : 4 v/v) to give Compound 2 (166 mg).

[Chemical Formula 6]

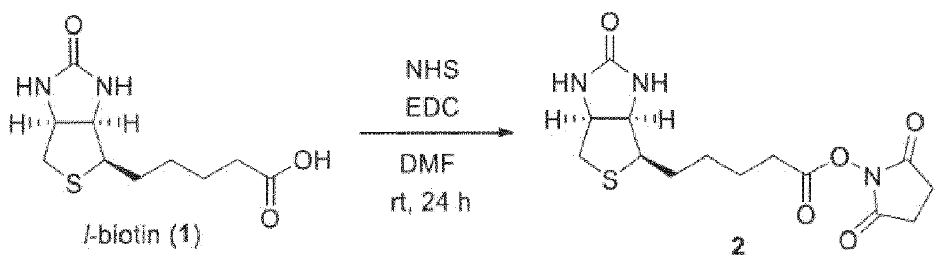


*l*-biotin (**1**)

**2**

6-(5-((3 aR,4R,6aS)-2-Oxohexahydro-1H-thieno [3,4-d]imidazol-4-yl)pentanamide)hexanoic acid (Compound 3) was synthesized using the Compound 2 according to the following synthesis scheme. Specifically, the synthesis was as follows. Compound 2 (166 mg, 0.458 mmol) was dissolved in DMF (2.6 mL). 6-Aminohexanoic acid (63.0 mg, 0.481 mmol) was dissolved in a 0.25 M aqueous sodium carbonate solution (1.0 mL). This solution was added to a DMF solution of Compound 2, and the mixture was stirred at room temperature for 24 hours.

The solvent was evaporated and the resulting residue was dissolved in water. The aqueous layer was acidified with 4 M hydrochloric acid at 0°C, and the precipitate was collected by filtration to give Compound 3 (159 mg).

[Chemical Formula 7]

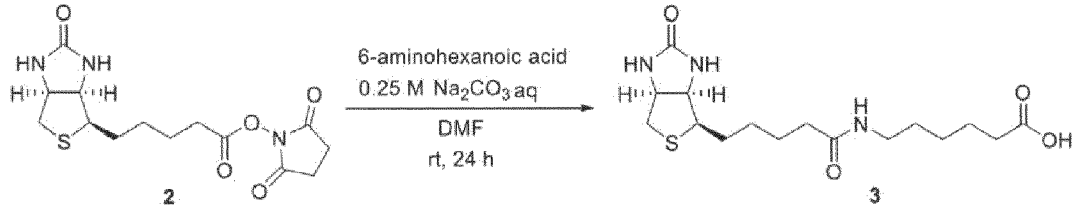


2 3

L-Biotin-AC5-NHS, that is, 2,5-dioxopyrrolidin-1-yl 6-(5-((3aR,4R,6aS)-2-oxohexahydro-1H-thieno[3,4-d]imidazol-4-yl)pentanamide) hexanoic acid ester (Compound 4) was synthesized using Compound 3 according to the following synthesis scheme. Specifically, the synthesis was as follows. Compound 3 (159 mg, 0.408 mmol) and NHS (93.9 mg, 0.816 mmol) were dissolved in DMF (7.6 mL), EDC (157 mg, 0.816 mmol) was added thereto, and the mixture was stirred at room temperature. The reaction mixture was stirred at 35°C for 23 hours. The solvent was evaporated and the resulting residue was purified by flash column chromatography ($CH_2Cl_2$/methanol (8 : 1 v/v)) to give Compound 4 (141 mg).

[Chemical Formula 8]

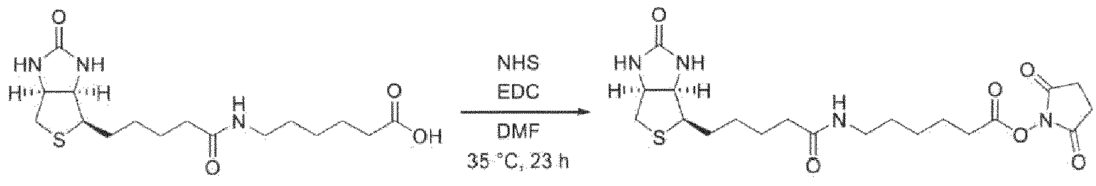


3 *l*-biotin-AC5-NHS (4)

The results of NMR analysis and mass spectrometry of Compound 4 were as follows. $^1$H NMR (400 MHz, DMSO-$d_6$) δ 7.75 (t, J = 1.8 Hz, 1H), 6.42 (s, 1H), 6.35 (s, 1H), 4.32-4.28 (m, 1H), 4.14-4.10 (m, 1H), 3.12-3.07 (m, 1H), 3.04-2.98 (m, 2H), 2.85-2.78 (m, 1H), 2.81 (s, 4H), 2.65 (t, J = 7.2 Hz, 2H), 2.57 (d, J = 12.8 Hz, 1H), 2.04 (t, J = 7.2 Hz, 2H), 1.65-1.22 (m, 12H). ESI-MS m/z 455.1967 [M+H]$^+$ (calcd for $C_{20}H_{31}N_4O_6S$, 455.1959).

(3.1.2) Synthesis of L-biotin-PE-maleimide

(3aR,4S,7R,7aS)-3a,4,7,7a-Tetrahydro-4,7-epoxyisobenzofuran-1,3-dione (Compound 7) was synthesized using maleic anhydride (Compound 5) and furan (Compound 6) according to the following synthesis scheme. Specifically, the synthesis was as follows. Maleic anhydride (4.0 g, 40.0 mmol) was dissolved in ethyl acetate (20.0 mL), furan (4.0 mL, 63.0 mmol) was added thereto, and the mixture was vigorously stirred. The reaction mixture was stirred at room temperature for 48 hours. The precipitate was collected by filtration and washed with ethyl acetate to give Compound 7 (4.03 g).

[Chemical Formula 9]

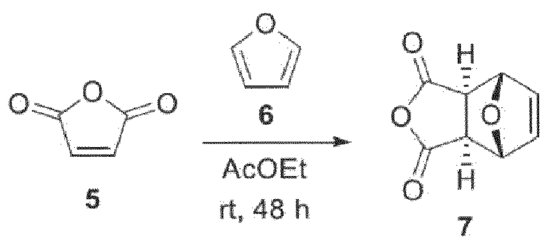


5 7

(3 aR,4 S, 7R,7aS)-2-(2-(Piperazin-1-yl)ethyl)-3 a, 4,7, 7a-tetrahydro-1H-4, 7-epoxyisoindol-1,3-(2H)dione (Compound 9) was synthesized using Compound 7 and N-(2-aminoethyl)piperazine (Compound 8) according to the following synthesis scheme. Specifically, the synthesis was as follows. Compound 7 (600 mg, 3.61 mmol) was dissolved

in ethanol (6.0 mL), and Compound 8 (0.520 mL, 3.97 mmol), triethylamine (0.520 mL, 3.97 mmol) and ethanol (1.2 mL) were added, then the mixture was stirred at 0°C for 30 minutes. The reaction mixture was warmed to 70°C and stirred at 70°C for 15 hours. The solvent was evaporated and the resulting residue was dissolved in ethyl acetate. The organic layer was washed with water, dried over $Na_2SO_4$ and filtered through celite. The filtrate was concentrated to give Compound 9 (518 mg).

[Chemical Formula 10]

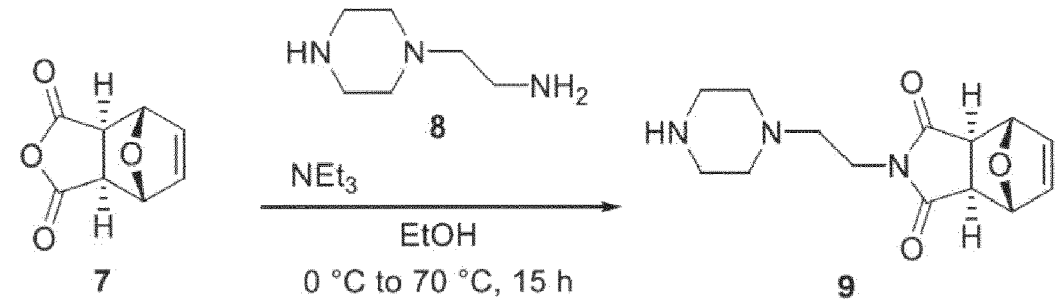


(3 aR,4 S, 7R,7aS)-2-(2-(4-(5-((3 aR,4R, 6aS)-2-Oxohexahydro-1H-thieno [3,4-d]imidazol-4-yl)pentanoyl)piperazin-1-yl)ethyl)-3a,4,7,7a-tetrahydro-1H-4,7-epoxyisoindol-1,3(2H)dione (Compound 10) was synthesized using L-biotin (Compound 1) and Compound 9 according to the following synthesis scheme. Specifically, the synthesis was as follows. L-Biotin (89.6 mg, 0.367 mmol), Compound 9 (123 mg, 0.442 mmol) and 4-(N,N-dimethylamino)pyridine (DMAP) (8.60 mg, 70.0 μmol) were dissolved in DMF (2.4 mL), EDC (84.7 mg, 0.442 mmol) was added thereto, and the mixture was stirred at room temperature for 15 hours. The solvent was evaporated and the resulting residue was purified by flash column chromatography ($CH_2Cl_2$/methanol (8 : 1 v/v)) to give Compound 10 (102 mg).

[Chemical Formula 11]

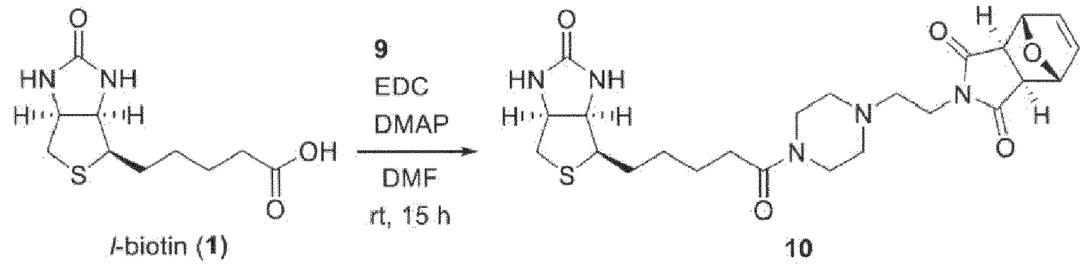


L-Biotin-PE-maleimide, that is, 1-2-(4-(5-((3aR,4R,6aS)-2-oxohexahydro-1H-thieno[3,4-d]imidazol-4-yl)pentanoyl)piperazin-1-yl)ethyl)-1H-pyrrole-2,5-dione (Compound 11) was synthesized using Compound 10 according to the following synthesis scheme. Specifically, the synthesis was as follows. Compound 10 (102 mg, 0.200 mmol) was dissolved in toluene (20.0 mL) and the solution was refluxed for 3 hours. The solvent was evaporated and the resulting residue was purified by flash column chromatography ($CH_2Cl_2$/methanol (8 : 1 v/v)) to give Compound 11 (70.0 mg).

[Chemical Formula 12]

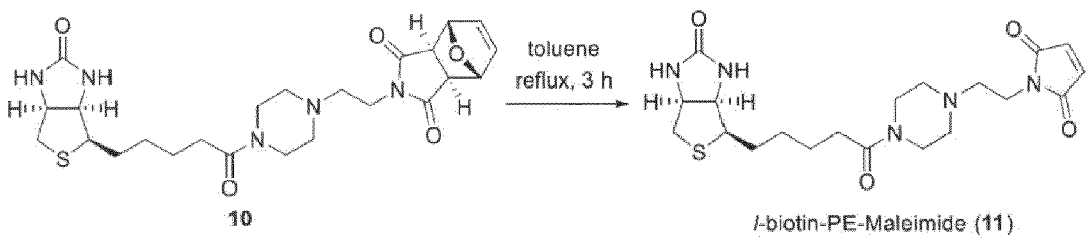


The results of NMR analysis and mass spectrometry of Compound 11 were as follows. $^1H$ NMR (400 MHz, DMSO-$d_6$) δ 7.01 (s, 2H), 6.42 (s, 1H), 6.34 (s, 1H), 4.28-4.24 (m, 1H), 4.11- 4.08 (m, 1H), 3.55 (t, J = 6.4 Hz, 2H),

3.37-3.26 (m, 4H), 3.08-3.03 (m, 1H), 2.80-2.76 (m, 1H), 2.58- 2.52 (m, 1H), 2.43 (t, J = 6.4 Hz, 2H), 2.34-2.32 (m, 2H), 2.28-2.26 (m, 2H), 2.29 (t, J = 7.6 Hz, 2H), 1.61-1.24 (m, 6H). $[\alpha]^{15}$ = -54.6° (C = 1.00, MeOH). ESI-MS m/z found 436.1995 $[M+H]^+$ (calcd for $C_{20}H_{31}N_4O_6S$, 436.2013).

(3.2) Preparation of sample containing biotin-labeled polypeptide

(3.2.1) Preparation of biotin-labeled BSA

L-Biotin-AC5-NHS (32.5 mg) was dissolved in DMF (0.65 mL) to obtain an L-biotin-AC5-NHS solution. BSA (5.63 g: Proliant Biologicals, LLC.) was dissolved in 0.1 M phosphate buffer (pH 7.5, 112.5 mL) to obtain a BSA solution. A BSA solution (0.65 mL) and an L-biotin-AC5-NHS solution (0.65 mL) were mixed, and the mixture was allowed to stand at 35°C for 1 hour. The reaction mixture was passed through a PD-10 column (Cytiva) equilibrated with 0.1 M phosphate buffer (pH 7.5), and fractions were collected in 500 μL portions. The absorbance of each fraction was measured, and a fraction with an absorption peak at 280 nm was used as a sample containing L-biotin-labeled BSA in subsequent experiments. A sample containing D-biotin labeled BSA was obtained in the same manner as described above except that D-biotin AC5-NHS was used in place of L-biotin AC5-NHS.

(3.2.2) Preparation of biotin-labeled antibody

L-Biotin-PE-maleimide (10 mg) was dissolved in dimethyl sulfoxide (DMSO) (1.7 mL) to obtain an L-biotin-PE-maleimide solution. From a mouse anti-thyroid stimulating hormone (TSH) monoclonal antibody (KIT AY AM A LABES CO., LTD.), an anti-TSH antibody F(ab')2 was obtained by a conventional method using pepsin. EDTA Disodium salt and sodium hydroxide were dissolved in ultrapure water to prepare a 0.1 M ethylenediaminetetraacetic acid (EDTA) solution. Phosphorylated monosodium dihydrate, EDTA disodium salt and sodium hydroxide were dissolved in ultrapure water to prepare a buffer for gel filtration. A 0.1 M EDTA solution and a buffer for gel filtration were added to F(ab')2 of the obtained anti-TSH antibody to obtain a solution having an antibody concentration of 8 mg/mL. A 0.3 M 2-Mercaptoethylamine (MEA) solution was added to this solution to reduce a disulfide bond of the antibody. The reaction mixture was passed through a PD-10 column (Cytiva) equilibrated with 0.1 M phosphate buffer (pH 7.5) to obtain a fraction with an absorption peak at 280 nm. A fraction containing the antibody (500 μL) and L-biotin-PE-maleimide (1.7 mL) were mixed, and the mixture was allowed to stand at 35°C for 1 hour. The reaction mixture was purified by ultrafiltration (cut-off molecular weight 30,000) and gel filtration column to obtain a solution of L-biotin-labeled anti-TSH antibody Fab'. The obtained solution was used as a sample containing an L-biotin-labeled antibody in the subsequent experiments. A sample containing a D-biotin-labeled antibody was obtained in the same manner as described above except that D-biotin-PE-maleimide was used in place of L-biotin-PE-maleimide.

4. Measurement of sample

(4.1) Preparation of calibration curve

A reagent for measuring L-biotin (91.7 μL) was mixed with 0.1 M phosphate buffer (pH 7.5, 18.3 μL) or an L-biotin standard solution (18.3 μL), and the mixture was allowed to stand at room temperature for 5 minutes. Then, the absorbance at 500 nm ($A_{500}$) of the reaction mixture was measured by spectrophotometer UV-2600 (Shimadzu Corporation). Measurement was performed in the same manner using Reagents 1 and 2 for measuring D-biotin and a D-biotin standard solution. As a calibration curve, a straight line by a least squares method was used. The measurement results of the absorbance are shown in Table 1. Examples of calibration curves for each measurement reagent are shown in Figs. 4A to 4D.

[Table 1]

| D-Biotin (μM) | $A_{500}$ | |
|---|---|---|
| | Measurement reagent 1 | Measurement reagent 2 |
| 0 | 0.334 | 0.221 |
| 25 | 0.194 | 0.156 |
| 50 | 0.062 | 0.090 |

(continued)

| L-Biotin (μM) | $A_{500}$ |
|---|---|
| 0 | 0.315 |
| 25 | 0.264 |
| 50 | 0.216 |

As shown in Table 1, Fig. 4A and Fig. 4B, the complex of avidin or streptavidin and HABA had an absorption at a wavelength of 500 nm, and the absorbance decreased depending on the D-biotin concentration of the standard solution. The coefficient of determination ($R^2$) of the calibration curve was 0.9997, and a good correlation was observed between the D-biotin concentration and the absorbance. Therefore, Reagents 1 and 2 for measuring D-biotin could reproduce commercially available reagents for biotin quantification as shown in Pierce (trademark) Biotin Quantitation Kit (product number: 28005), Thermo Fisher Scientific. Referring to Table 1, Fig. 4C and Fig. 4D, the complex of optically isomeric streptavidin and HABA also had an absorption at a wavelength of 500 nm, and the absorbance decreased depending on the L-biotin concentration of the standard solution. The coefficient of determination of the calibration curve for the reagent for measuring L-biotin was also 0.9997, and a good correlation was observed between the L-biotin concentration and the absorbance. Therefore, it was shown that the concentration of free L-biotin in the sample can be determined by a reagent containing optically isomeric streptavidin and HABA.

(4.2) Determination of biotin concentration in sample

A sample containing the L-biotin-labeled BSA prepared in the above (3.2.1) was diluted 100-fold with 0.1 M phosphate buffer (pH 7.5). A reagent for measuring L-biotin (91.7 μL) and a sample containing diluted L-biotin-labeled BSA (18.3 μL) or a sample containing an L-biotin-labeled antibody (18.3 μL) were mixed, and the mixture was allowed to stand at room temperature for 5 minutes. Then, $A_{500}$ of the reaction mixture was measured by UV-2600. The amount (μM) of L-biotin contained in each sample was determined from a calibration curve (regression equation) for the reagent for measuring L-biotin. Measurement was performed in the same manner using Reagent 1 for measuring D-biotin and a sample containing D-biotin-labeled BSA or a D-biotin-labeled antibody, and the concentration (μM) of D-biotin contained in each sample was determined from the calibration curve. The measurement results of $A_{500}$ and biotin concentration of each sample are shown in Table 2. As the biotin concentration of the sample containing the L-biotin-labeled BSA and the D-biotin-labeled BSA, a value obtained by multiplying the value determined from the calibration curve by 100 was used.

[Table 2]

| Sample | $A_{500}$ | D-Biotin (μM) |
|---|---|---|
| D-Bio-BSA | 0.257 | 1375.0 |
| D-Bio-Fab' | 0.068 | 47.5 |
| Sample | $A_{500}$ | L-Biotin (μM) |
| L-Bio-BSA | 0.234 | 1231.9 |
| L-Bio-Fab' | 0.232 | 81.4 |

As can be seen from Table 2, the D-biotin concentration in the sample containing each of the D-biotin-labeled BSA and the D-biotin-labeled antibody could be measured by Reagent 1 for measuring D-biotin. The L-biotin concentration in the sample containing L-biotin-labeled BSA and the sample containing an L-biotin-labeled antibody could be measured by the reagent for measuring L-biotin, similarly to the reagent for measuring D-biotin. Therefore, it was suggested that the concentration of the bound L-biotin in the sample can be measured by the reagent for measuring L-biotin, similarly to the reagent for measuring D-biotin.

5. Determination of number of biotin groups of biotin-labeled polypeptide

In order to determine the protein concentration (concentration of BSA or Fab') of the above samples, the absorbance ($A_{280}$) of each sample at 280 nm was measured by spectrophotometer NanoDrop (trademark) (Thermo Fisher Scientific). With $A_{280}$ of the BSA solution (1 mg/mL) as 0.63, the BSA concentration (mg/mL) in each sample was calculated from the measured values of $A_{280}$ of the sample containing D-biotin-labeled BSA and the sample containing

L-biotin-labeled BSA. The BSA concentration (mg/mL) in each sample was converted to a molar concentration (μM) with the molecular weight of BSA as 66200. Similarly, with $A_{280}$ of the Fab' solution (1 mg/mL) as 1.38, the Fab' concentration (mg/mL) in each sample was calculated from the measured values of $A_{280}$ of the sample containing a D-biotin-labeled antibody and the sample containing an L-biotin-labeled antibody. The Fab' concentration (mg/mL) in each sample was converted to a molar concentration (μM) with the molecular weight of Fab' as 46000. For each sample, the number of biotin groups of one molecule of the biotin-labeled polypeptide (hereinafter, also referred to as the number of labels) was calculated by dividing the biotin concentration determined in the above 4. by the molar concentration of BSA or Fab'. The results are shown in Table 3.

[Table 3]

| Sample | Biotin concentration (μM) | BSA concentration (μM) | Fab' concentration (μM) | Number of labels |
|---|---|---|---|---|
| D-Bio-BSA | 1375.0 | 151.05 | - | 9.1 |
| D-Bio-Fab' | 47.5 | - | 14.78 | 3.2 |
| L-Bio-BSA | 1231.9 | 151.05 | - | 8.2 |
| L-Bio-Fab' | 81.4 | - | 23.91 | 3.4 |

As shown in Table 3, the number of D-biotin groups of the D-biotin labeled polypeptide could be determined from the D-biotin concentration in the sample determined by Reagent 1 for measuring D-biotin and the protein concentration in the sample. Similarly, the number of L-biotin groups of the L-biotin-labeled polypeptide could be determined from the L-biotin concentration in the sample determined by the reagent for measuring L-biotin and the protein concentration in the sample. Therefore, it was suggested that the number of labels of the L-biotin labeling substance in the sample can be measured by the reagent for measuring L-biotin, similarly to the reagent for measuring D-biotin.

Example 2: Production and Use of Reagent Kit for Immunological Measurement

A reagent kit for immunological measurement was produced using L-biotin-labeled BSA and L-biotin-labeled antibody that were confirmed to satisfy the standard value of the number of labels in Example 1. TSH in the sample was measured using the reagent kit.

1. Production of solid phase on which optically isomeric streptavidin is immobilized

The magnetic particle MAG2201 (2 g: JSR Corporation) was washed with 20 mM phosphate buffer (pH 6.0, 4 mL). The magnetic particle had a carboxyl group as a functional group on the surface. A solution of an activator to convert the carboxyl group to an NHS ester was prepared by dissolving N-hydroxysuccinimide (6.2 g) and carbodiimide (19.2 g) in 20 mM phosphate buffer (pH 6.0, 2000 mL). The magnetic particle was magnetically separated to remove the supernatant, the solution (4 mL) of an activator was added thereto, and the mixture was stirred at 300 rpm at 15°C to 30°C for 15 minutes. The magnetic particle was magnetically separated to remove the supernatant, and 20 mM phosphate buffer (pH 6.0, 4 mL) was added to wash the magnetic particle. The magnetic particle was magnetically separated to remove the supernatant, 20 mM phosphate buffer (pH 7.5, 3.6 mL) was added, and the mixture was stirred. The L-biotin-labeled albumin (10 mg/mL, 0.4 mL) prepared in Example 1 was added to the suspension of magnetic particles, and the mixture was stirred at 300 rpm at 15°C to 30°C for 120 minutes. As a result, the NHS ester on the magnetic particle was bound to the albumin portion of L-biotin-labeled albumin, and the surface of the magnetic particle was labeled with biotin. The magnetic particle was magnetically separated to remove the supernatant, and 20 mM phosphate buffer (pH 7.5, 3.6 mL) was added to wash the magnetic particle. To the magnetic particle was added 20 mM phosphate buffer (pH 7.5, 2 mL). Optically isomeric streptavidin (544 mg) was dissolved in 20 mM phosphate buffer (pH 6.0, 1000 mL) to obtain an optically isomeric streptavidin solution. An optically isomeric streptavidin solution (3.6 mL) was added to the suspension of magnetic particles, and the mixture was stirred at 300 rpm at 15°C to 30°C for 10 minutes. As a result, L-biotin on the magnetic particle bound to the optically isomeric streptavidin, and the optically isomeric streptavidin was immobilized on the surface of the magnetic particle. The magnetic particle was magnetically separated to remove the supernatant, and buffer for storing a magnetic particle (MES buffer (pH 6.5)) was added to wash the magnetic particle. The buffer for storing a magnetic particle was added to the magnetic particle to obtain a magnetic particle (18 mL) on which the optically isomeric streptavidin was immobilized.

2. Preparation of each reagent of reagent kit for immunological measurement

As an R1 reagent (capture antibody reagent), the solution of the L-biotin-labeled anti-TSH antibody Fab' prepared in Example 1 was used. As an R2 reagent (solid phase), the suspension of magnetic particles on which the optically isomeric streptavidin prepared in the above 1. was immobilized was used. As an R3 reagent (detection antibody reagent), a HISCL (trademark) TSH R3 reagent (Sysmex Corporation) containing an ALP-labeled mouse anti-TSH antibody monoclonal antibody was used. As an R4 reagent (measurement buffer), a HISCL R4 reagent (Sysmex Corporation) was used. As an R5 reagent (substrate solution), a HISCL R5 reagent (Sysmex Corporation) containing CDP-Star (trademark) was used. As buffer for dilution, a HISCL specimen diluent (Sysmex Corporation) was used. A HISCL washing solution (Sysmex Corporation) was used as a washing solution. For comparison, a HISCL TSH reagent (Sysmex Corporation), which is a commercially available kit for measuring TSH, was used. This reagent included a HISCL TSH R1 reagent containing a D-biotin-labeled anti-TSH antibody, a HISCL TSH R2 reagent containing a magnetic particle on which streptavidin was immobilized, a HISCL (trademark) TSH R3 reagent, a HISCL R4 reagent, and a HISCL R5 reagent.

3. Measurement of TSH

As a sample, a HISCL TSH calibrator (Sysmex Corporation) was used. The calibrator is a kit composed of six samples containing six different concentrations of TSH. TSH in each sample was measured by a fully automated immunoassay device HISCL-5000 (Sysmex Corporation). Specific operations are as follows. The R1 reagent (30 μL) and diluted serum (30 μL) were mixed and incubated at 42°C for 2 minutes. The R2 reagent (30 μL) was added to the reaction mixture, mixed, and incubated at 42°C for 2.5 minutes. The R3 reagent (30 μL) was added to the reaction mixture, and the mixture was mixed and incubated at 42°C for 2.5 minutes. Thereafter, the magnetic particle was magnetically separated to remove the supernatant, and a washing solution (300 μL) was added to wash the magnetic particle. The magnetic particle was washed four times. The supernatant was removed, and the R4 reagent (50 μL) was added to the magnetic particle and mixed, then the R5 reagent (50 μL) was added thereto, and the mixture was mixed and incubated at 42°C for 5 minutes. Then, the emission intensity of the reaction mixture was measured. For comparison, TSH was measured using a HISCL TSH reagent and HISCL-5000 according to the attached documents of the reagent. Each sample constituting the calibrator was continuously measured three times. An average value of the light emission count values obtained by three measurements was determined and used as a measured value.

4. Results

Fig. 5 shows a graph obtained by plotting measured values obtained by measuring the calibrator using the reagent kit of Example 2 including the L-biotin-labeled anti-TSH antibody Fab' and the magnetic particle on which the optically isomeric streptavidin was immobilized and measured values using the HISCL TSH reagent As can be seen from Fig. 5

## Claims

1. A reagent for measuring L-biotin, comprising an optically isomeric biotin-binding site and an azobenzene derivative represented by following formula (I):

[Chemical Formula 1]

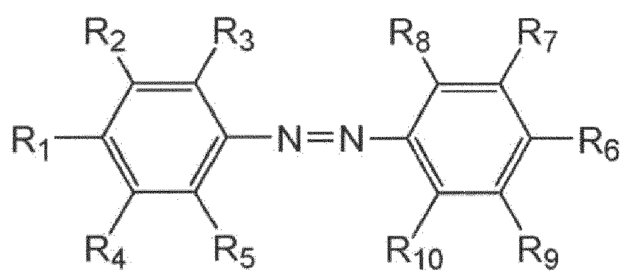

(I)

wherein $R_1$ to $R_{10}$ are each independently a group selected from the group consisting of a hydrogen atom, a hydroxy

group, a carboxy group, C1 to C6 dialkylamino groups having no substituent or a substituted group, C1 to C6 alkyl groups having no substituent or a substituted group, and C1 to C6 alkoxy groups having no substituent or a substituted group, provided that at least one of $R_1$ to $R_5$ is a hydroxy group or a C1 to C6 dialkylamino group having no substituent or a substituted group, and at least one of $R_6$ to $R_{10}$ is a carboxy group.

**2.** The reagent according to claim 1, wherein the optically isomeric biotin-binding site is a polypeptide that has a part or all of an amino acid sequence of an L-type biotin-binding site, 90% or more of amino acid residues other than glycine in the amino acid sequence being D-amino acid residues, binds to L-biotin, and does not substantially bind to D-biotin.

**3.** The reagent according to claim 1 or 2, wherein the optically isomeric biotin-binding site is at least one selected from the group consisting of optically isomeric streptavidin, optically isomeric streptavidin variants, optically isomeric avidins, optically isomeric tamavidins, optically isomeric bradavidins, and optically isomeric rhizavidins.

**4.** The reagent according to any one of claims 1 to 3, wherein the optically isomeric biotin-binding site is a polypeptide in which the amino acid residues other than glycine comprise D-amino acid residues.

**5.** The reagent according to any one of claims 1 to 4, wherein the azobenzene derivative is 4'-hydroxyazobenzene-2-carboxylic acid, 4'-hydroxyazobenzene-4-carboxylic acid, or 4'-dimethylaminoazobenzene-2-carboxylic acid.

**6.** A method for measuring a sample comprising L-biotin, comprising:

preparing a measurement sample by mixing a sample comprising L-biotin with the reagent for measuring L-biotin according to any one of claims 1 to 5; and
measuring absorbance of the measurement sample,
the measured value of absorbance being an index of concentration of L-biotin in the sample.

**7.** The measurement method according to claim 6, further comprising determining a concentration of L-biotin in the sample based on the measured value of absorbance.

**8.** A method for determining a number of labels of an L-biotin-labeled substance, comprising:

preparing a measurement sample by mixing a sample comprising an L-biotin-labeled substance with the reagent for measuring L-biotin according to any one of claims 1 to 5;
measuring absorbance of the measurement sample;
determining a concentration of L-biotin in the sample based on the measured value of absorbance; and
determining a number of L-biotin groups per molecule of the L-biotin-labeled substance based on the concentration of L-biotin in the sample and a concentration of the substance.

**9.** The determination method according to claim 8, further comprising determining the concentration of the substance in the sample.

**10.** The determination method according to claim 8 or 9, the number of L-biotin groups per molecule of the L-biotin-labeled substance being calculated by following formula:

$$X = A/B$$

wherein X is the number of L-biotin groups per molecule of the L-biotin-labeled substance,
A is a molar concentration of L-biotin in the sample, and
B is a molar concentration of the substance in the sample.

**11.** The method according to any one of claims 8 to 10, the L-biotin-labeled substance being an L-biotin-labeled polypeptide.

**12.** The method according to claim 11, the L-biotin-labeled polypeptide being an L-biotin-labeled antibody.

**13.** A method for producing a solid phase on which an optically isomeric biotin-binding site is immobilized, comprising:

preparing a measurement sample by mixing a sample separated from a liquid comprising an L-biotin-labeled polypeptide with the reagent for measuring L-biotin according to any one of claims 1 to 5;
measuring absorbance of the measurement sample;
determining a concentration of L-biotin in the measurement sample based on the measured value of absorbance;
determining a number of L-biotin groups per molecule of the L-biotin-labeled polypeptide in the sample based on the concentration of L-biotin and a concentration of the polypeptide;
when the number of L-biotin groups is within a predetermined range, contacting the liquid with a solid phase capable of binding to the polypeptide to immobilize the L-biotin-labeled polypeptide on the solid phase; and
contacting the solid phase on which the L-biotin-labeled polypeptide is immobilized with an optically isomeric biotin-binding site to immobilize the optically isomeric biotin-binding site on the solid phase.

**14.** The method according to claim 13, further comprising determining the concentration of the polypeptide in the sample.

**15.** The method according to claim 13 or 14, the number of L-biotin groups per molecule of the L-biotin-labeled substance being calculated by following formula:

$$X = A/B$$

wherein X is the number of L-biotin groups per molecule of the L-biotin-labeled substance,
A is a molar concentration of L-biotin in the sample, and
B is a molar concentration of the substance in the sample.

**16.** The method according to any one of claims 13 to 15, the L-biotin-labeled polypeptide being L-biotin-labeled albumin.

FIG. 1A

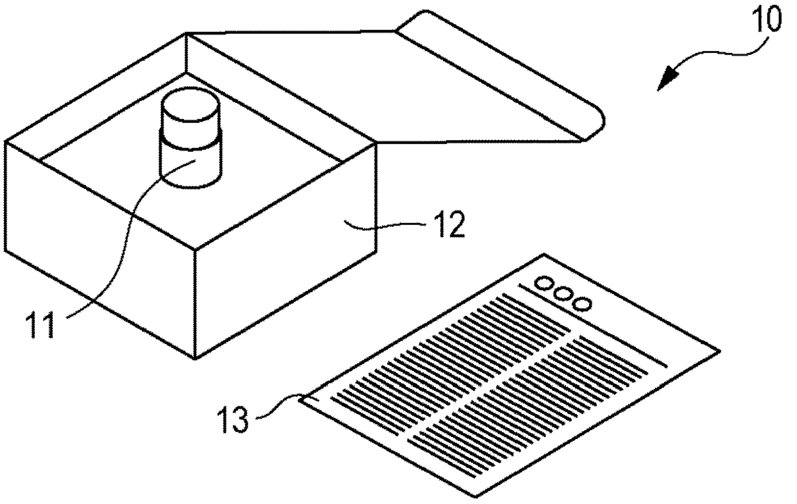


FIG. 1B

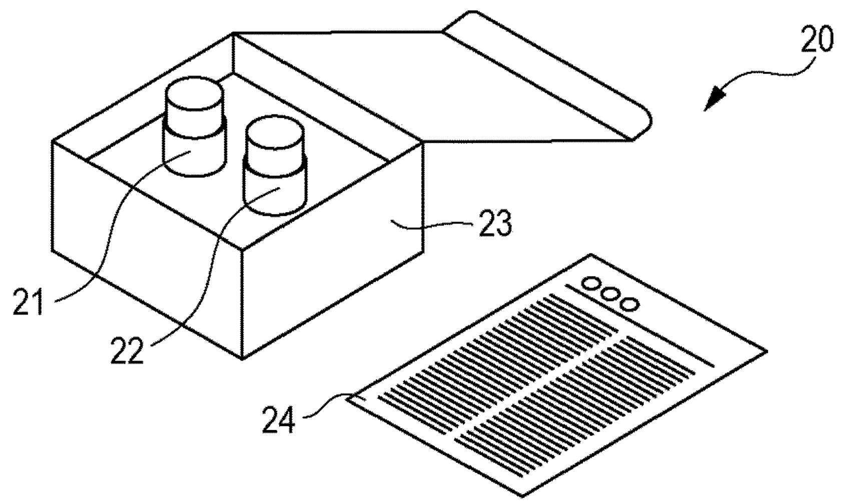

FIG. 2

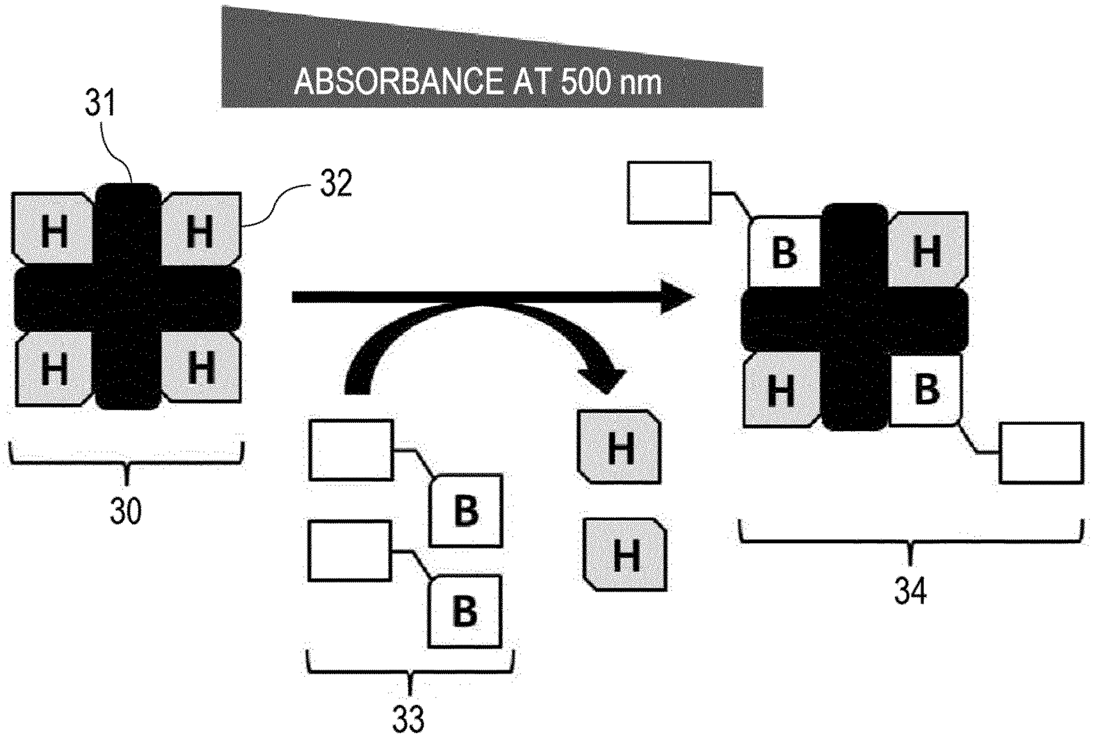
ABSORBANCE AT 500 nm
31
32
H
H
H
H
30
B
B
33
H
H
B
H
H
B
34

FIG. 3

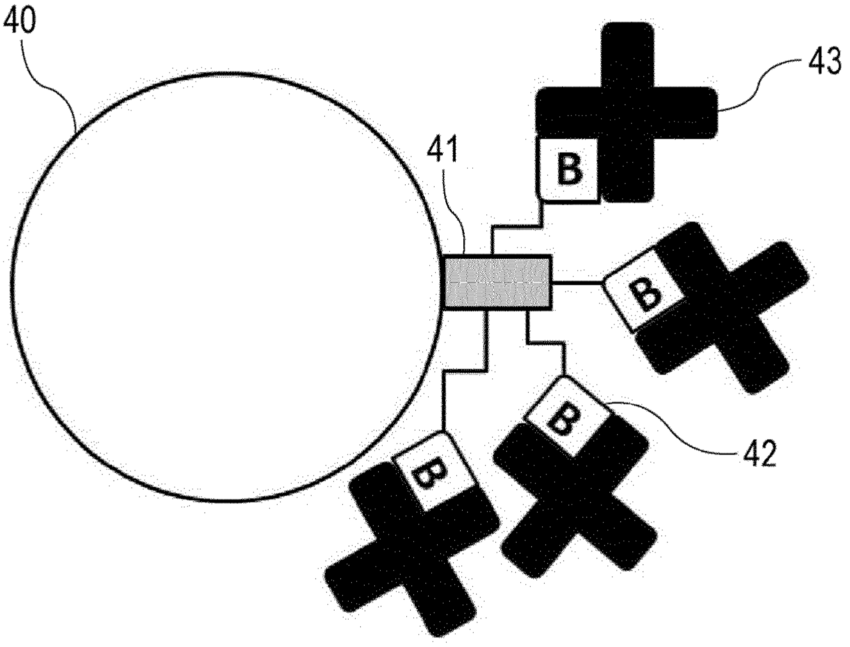
40
43
41
B
B
B
B
42

FIG. 4A

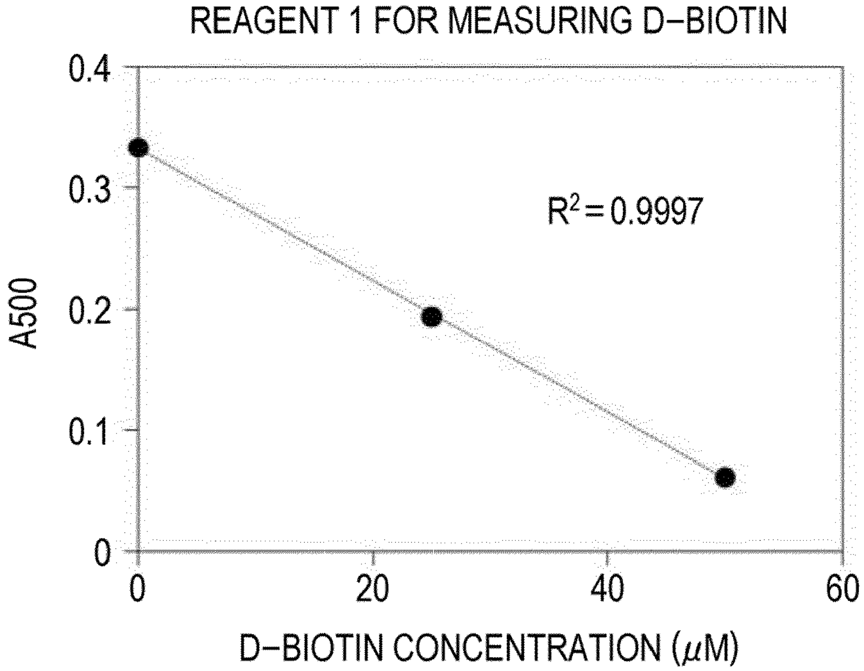
REAGENT 1 FOR MEASURING D−BIOTIN
0.4
0.3
0.2
0.1
0
A500
R2 = 0.9997
0
20
40
60
D−BIOTIN CONCENTRATION (μM)

FIG. 4B

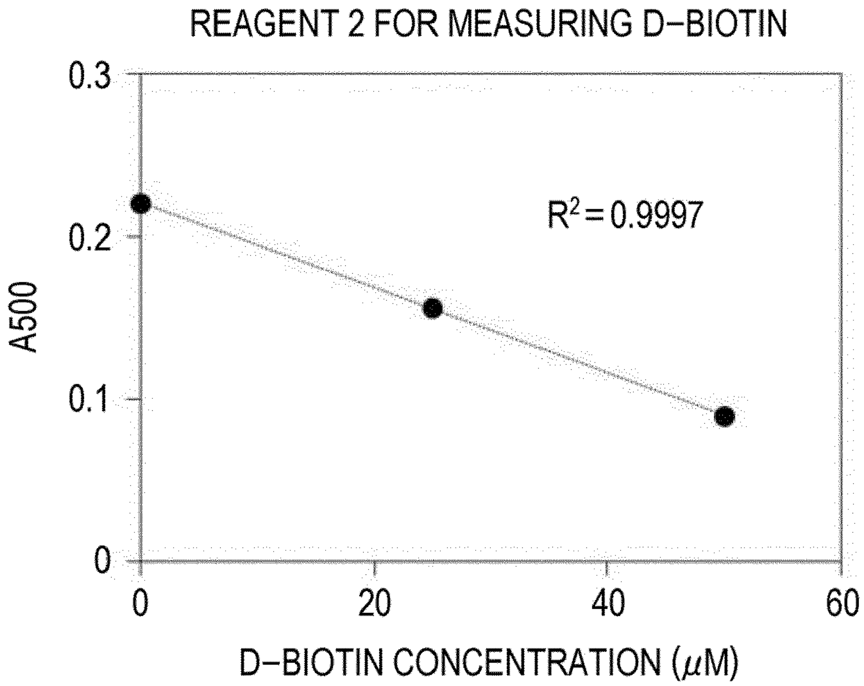
REAGENT 2 FOR MEASURING D−BIOTIN
0.3
0.2
0.1
0
A500
R2 = 0.9997
0
20
40
60
D−BIOTIN CONCENTRATION (μM)

*FIG. 4C*

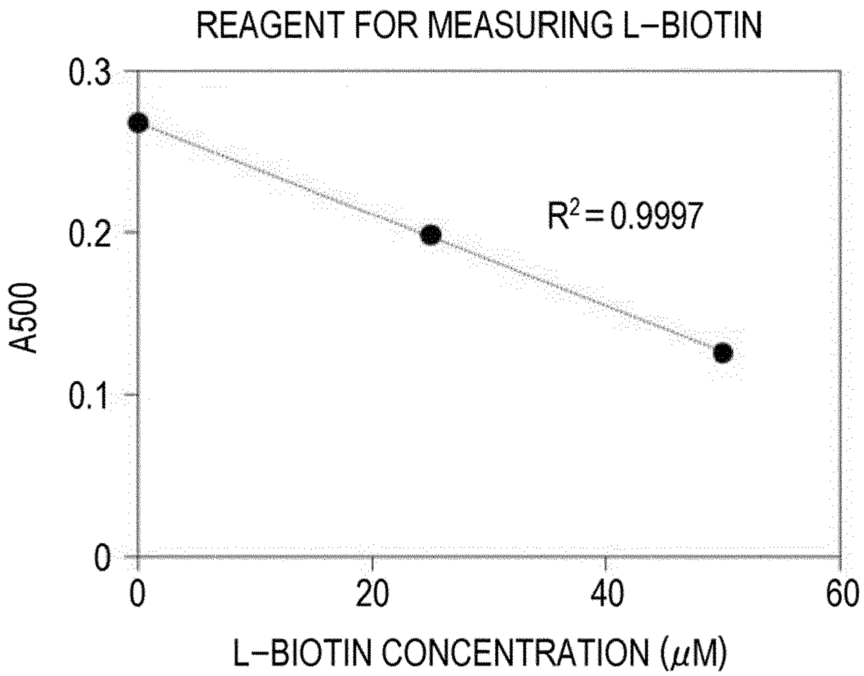
REAGENT FOR MEASURING L−BIOTIN
A500
0.3
0.2
0.1
0
R2 = 0.9997
0
20
40
60
L−BIOTIN CONCENTRATION (μM)

*FIG. 4D*

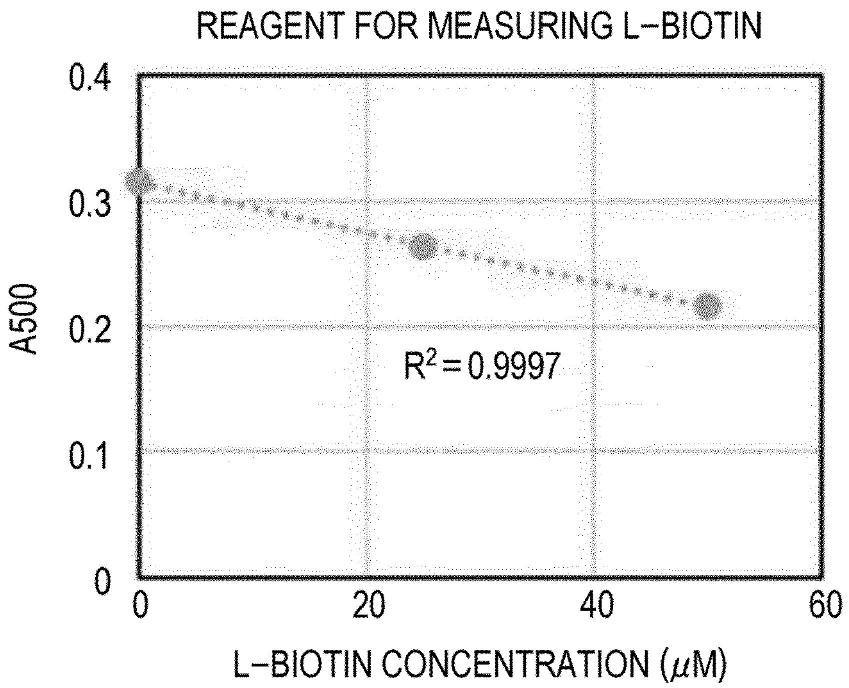
REAGENT FOR MEASURING L−BIOTIN
A500
0.4
0.3
0.2
0.1
0
R2 = 0.9997
0
20
40
60
L−BIOTIN CONCENTRATION (μM)

FIG. 5

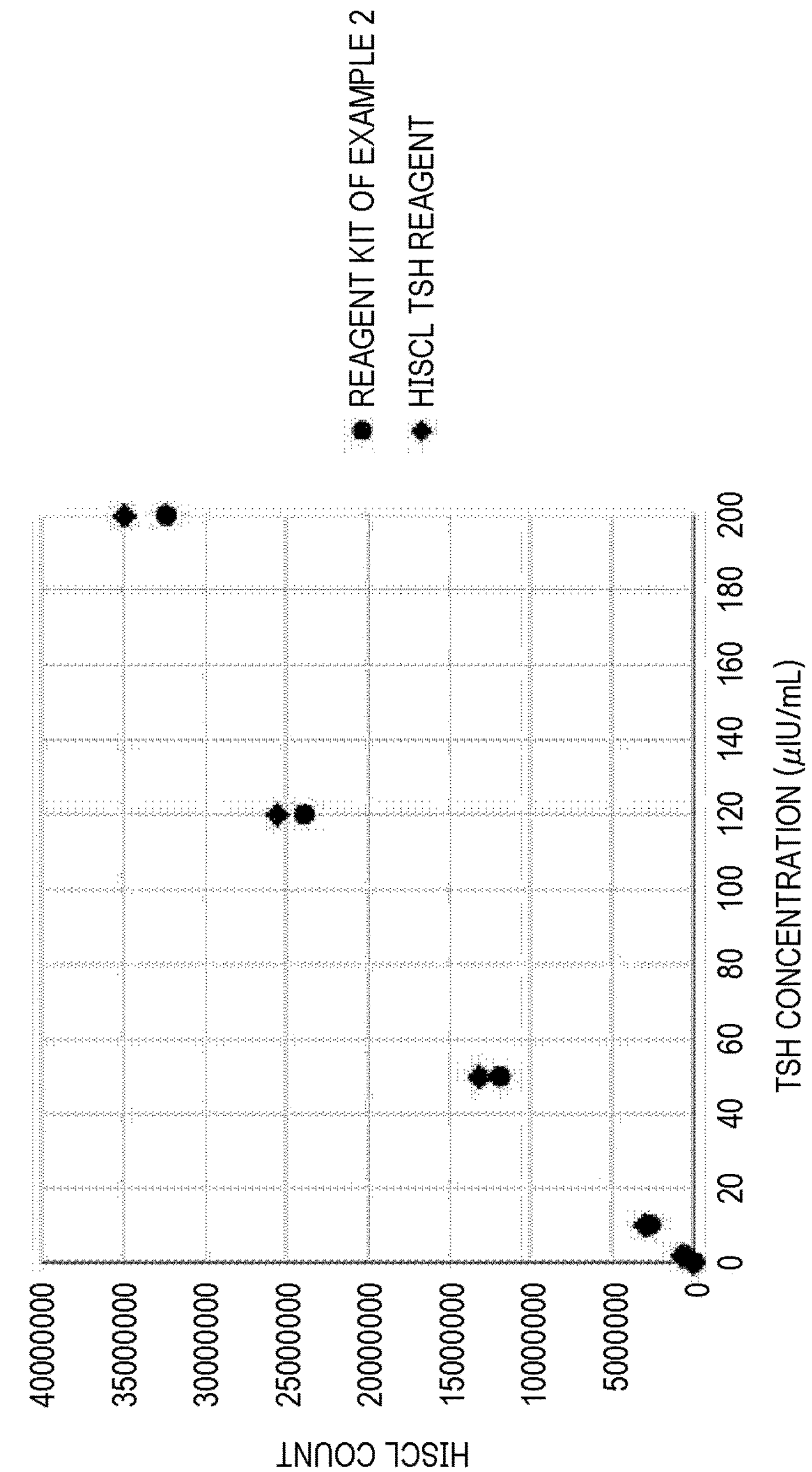
REAGENT KIT OF EXAMPLE 2
HISCL TSH REAGENT
HISCL COUNT
40000000
35000000
30000000
25000000
20000000
15000000
10000000
5000000
0
0
20
40
60
80
100
120
140
160
180
200
TSH CONCENTRATION (μIU/mL)

Europäisches Patentamt
European Patent Office
Office européen des brevets

# EUROPEAN SEARCH REPORT

Application Number
EP 23 16 0420

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 416 738 B1 (THEODORE LOUIS J [US] ET AL) 9 July 2002 (2002-07-09)<br>* column 50, last paragraph - column 51, paragraph 1 *<br>----- | 1-16 | INV.<br>G01N33/58<br>G01N33/53 |
| Y | BERG LUECKE LINDA ET AL: "Assessment of Streptavidin Bead Binding Capacity to Improve Quality of Streptavidin-based Enrichment Studies",<br>JOURNAL OF PROTEOME RESEARCH,<br>vol. 20, no. 2,<br>5 February 2021 (2021-02-05), pages 1153-1164, XP055851712,<br>* figure 3 *<br>----- | 1-16 | |
| A,P | SUGANUMA MASATOSHI ET AL: "Mirror-image streptavidin with specific binding to L-biotin, the unnatural enantiomer",<br>SCIENTIFIC REPORTS,<br>vol. 12, no. 1, 10 June 2022 (2022-06-10), page 9568, XP093067860,<br>* the whole document *<br>----- | 1-16 | TECHNICAL FIELDS SEARCHED (IPC)<br><br>G01N |
| A,P | WO 2022/080486 A1 (SYSMEX CORP [JP])<br>21 April 2022 (2022-04-21)<br>* the whole document *<br>----- | 1-16 | |
| A | MILTON R ET AL: "Total Chemical Synthesis of a D-Enzyme: The Enantiomers of HIV-1 Protease Show Demonstration of Reciprocal Chiral Substrate Specificity",<br>SCIENCE,<br>vol. 256, 1992, pages 1445-1448, XP093068627,<br>* the whole document *<br>-----<br>-/-- | 1-16 | |

The present search report has been drawn up for all claims

1

| Place of search | Date of completion of the search | Examiner |
|---|---|---|
| The Hague | 30 July 2023 | Gunster, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 23 16 0420

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHAO LE ET AL: "Mirror image proteins", CURRENT OPINION IN CHEMICAL BIOLOGY, vol. 22, 2014, pages 56-61, XP055923423, * the whole document * ----- | 1-16 | |
| A | US 6 908 903 B1 (THEODORE LOUIS J [US] ET AL) 21 June 2005 (2005-06-21) * column 14, line 44 - line 46 * ----- | 1-16 | |
| A | LUONG JOHN H T ET AL: "Biotin interference in immunoassays based on biotin-strept(avidin) chemistry: An emerging threat", BIOTECHNOLOGY ADVANCES, ELSEVIER PUBLISHING, BARKING, GB, vol. 37, no. 5, 2019, pages 634-641, XP085733152, * the whole document * ----- | 1-16 | |
| | | | TECHNICAL FIELDS SEARCHED (IPC) |

1

The present search report has been drawn up for all claims

| Place of search | Date of completion of the search | Examiner |
|---|---|---|
| The Hague | 30 July 2023 | Gunster, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 16 0420

This annex lists the patent family members relating to the patent documents cited in the above-mentioned European search report. The members are as contained in the European Patent Office EDP file on
The European Patent Office is in no way liable for these particulars which are merely given for the purpose of information.

30-07-2023

| Patent document cited in search report | | Publication date | Patent family member(s) | | Publication date |
|---|---|---|---|---|---|
| US 6416738 | B1 | 09-07-2002 | NONE | | |
| WO 2022080486 | A1 | 21-04-2022 | CN 116348498 | A | 27-06-2023 |
| | | | EP 4230643 | A1 | 23-08-2023 |
| | | | JP WO2022080486 | A1 | 21-04-2022 |
| | | | WO 2022080486 | A1 | 21-04-2022 |
| US 6908903 | B1 | 21-06-2005 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2002072817 A
- WO 2006058226 A


### Non-patent literature cited in the description


- **QURESHI MH. et al.** *J. Biol. Chem.,* 2001, vol. 276 (49), 46422-46428
- **WU SC. ; WONG SL.** *J. Biol. Chem.,* 2005, vol. 280 (24), 23225-23231
- **LIM KH. et al.** *Biotech. Bioeng.,* 2013, vol. 110 (1), 57-67
- **SANO T. et al.** *Proc. Natl. Acad. Sci. USA,* 1997, vol. 94, 6153-6158
- **DAWSON PE et al.** *Science,* 1994, vol. 266, 776-779
- **YAN LZ ; DAWSON PE.** *J. Am. Chem. Soc.,* 2001, vol. 123, 526-533
- **ARAKAWA T. ; EJIMA D.** *Antibodies,* 2014, vol. 3, 232-241
- **LAVIELLE S. et al.** *J. Am. Chem. Soc.,* 1978, vol. 100, 1558-1563